# FASCICULE DE BREVET EUROPEEN

(11) **EP 0 968 297 B1**
(45) Date de publication et mention de la délivrance du brevet: **23.03.2005**
(21) Numéro de dépôt: 98904207.2
(22) Date de dépôt: 23.01.1998
(51) Int. Cl.: C12N 15/86, A61K 35/76, C12N 15/38, C12N 7/01

(54) **VACCIN VIVANT RECOMBINANT AVIAIRE, UTILISANT COMME VECTEUR LE VIRUS DE LA LARYNGOTRACHEITE INFECTIEUSE AVIAIRE**
LEBENDER, REKOMBINANTER VOGELIMPFSTOFF UNTER VERWENDUNG DES INFEKTIÖSEN LARYNGOTRACHEITIS-VIRUS ALS VEKTOR
LIVE RECOMBINANT AVIAN VACCINE USING THE AVIAN INFECTIOUS LARYNGOTRACHEITIS VIRUS AS VECTOR

(30) Priorité: 31.01.1997 FR 9701317
(43) Date de publication de la demande: 05.01.2000
(73) Titulaire: MERIAL, 69002 Lyon (FR)
(72) Inventeur: BUBLOT, Michel, F-69290 Saint Genis les Olières (FR); LAPLACE, Eliane, F-69600 Oullins (FR); AUDONNET, Jean-Christophe, F-69006 Lyon (FR)
(74) Mandataire: Nargolwalla, Cyra
(86) Numéro de dépôt international: PCT/FR1998/000122
(87) Numéro de publication internationale: WO 1998/033928

(56) Documents cités:
- EP-A- 0 719 864
- WO-A-92/03554
- WO-A-96/00791

## Description

La présente invention a trait à des vaccins à usage aviaire à base de virus de la laryngotrachéite infectieuse (ILTV), dans lequel a été insérée, par recombinaison génétique, au moins une séquence nucléotidique hétérologue, notamment codant pour, et exprimant, un polypeptide antigénique d'un agent pathogène aviaire, dans des conditions assurant une immunisation conduisant à une protection efficace de l'animal vacciné contre ledit agent pathogène.

Le virus de la laryngotrachéite infectieuse (ILTV) est un alphaherpèsvirus (B. Roizman, *Arch. Virol* 1992. **123**. 425-449) qui provoque une pathologie respiratoire importante (la laryngotrachéite infectieuse ou ILT) chez le poulet (L.E. Hanson et T.J. Bagust, *Diseases of Poultry* 9th edn 1991.pp 485-495. Ames, Iowa State University Press). Les vaccins actuellement disponibles contre cette affection contiennent une souche atténuée administrable par différentes voies dont les voies intranasales, conjonctivales, cloacales, dans l'eau de boisson et par aérosol (L.E. Hanson et T.J. Bagust, *Diseases of Poultry* 9th Edition 1991.pp 485-495. Ames, Iowa State University Press).

Les études de biologie moléculaire du virus ILTV ont permis de caractériser le génome viral (M.A. Johnson *et al., Arch*. *Virol*, 1991. **119.** 181-198) et d'identifier quelques gènes du virus (A.M. Griffin, *J. Gen. Virol*. 1989. **70**. 3085-3089) dont les gènes codant pour la thymidine kinase (UL23) (A.M. Griffin et M.E.G. Boursnell, *J. Gen. Virol*. 1990. **71**. 841-850; C.L. Keeler *et al., Avian Dis.* 1991. **35**. 920-929), la glycoprotéine gB (UL27) (A.M. Griffin, *J*. *Gen. Virol*. 1991. **72.** 393-398; K. Kongsuwan *et al*., *Virology* 1991. **184**. 404-410; D.J. Poulsen *et al., Virus Genes* 1991. **5**. 335-347), la glycoprotéine gC (UL44) (D.H. Kingsley *et al. , Virology* 1994. **203.** 336-343), la protéine de capside p40 (UL26) (A.M. Griffin, *Nucl. Acids Res.* 1990. **18**. 3664), la protéine homologue de la protéine ICP4 de l'herpès simplex (HSV-1) (M.A. Johnson *et al. , Virus Research* 1995. **35**. 193-204), les protéines homologues aux protéines ICP27 (UL54), glycoprotéine gK (UL53) et DNA hélicase (UL52) de l'HSV-1 (M.A. Johnson *et al*., *Arch. Virol*. 1995. **140**. 623-634), la ribonucléotide réductase (A.M. Griffin, *J*. *Gen. Virol*. 1989. **70.** 3085-3089, WO-A-90/02802), les gène UL1 à UL5 (W. Fuchs et T. C. Mettentleiter, *J. Gen. Virol*. 1996. **77**. 2221-2229), les gènes présents dans la séquence unique courte du génome (Uₛ) (M. A. Johnson *et al*., *DNA Sequence- The Journal of Sequencing and Mapping* 1995. **Vol. 5**. pp191-194; K. Kongsuwan *et al. , Arch. Virol*. 1995. **140**. 27-39; K. Kongsuwan *et al*., *Virus Research* 1993. **29**. 125-140; K. Kongsuwan *et al*., *Virus Gene* 1993. **7**. 297-303; M.A. Wild *et al*., *Virus Genes* 1996. **12**. 107-116; WO-A-92/03554; WO-A-95/08622).

La présente invention a pour objectif de mettre au point un vaccin aviaire à base de virus ILTV recombinant exprimant un gène hétérologue, ce virus étant capable de se répliquer et d'induire une immunité chez l'hôte vacciné tout en conservant une bonne innocuité.

Un autre objectif de l'invention est de proposer un tel vaccin qui soit en même temps particulièrement efficace contre la laryngotrachéite infectieuse (ILT).

Un autre objectif de l'invention est de proposer un tel vaccin qui soit utilisable dans la vaccination de masse par voie mucosale, par exemple par voie aérosol ou dans l'eau de boisson, de telle manière que la réplication du virus au niveau mucosal permette d'induire une immunité mucosale et systémique. Une telle immunité mucosale sera particulièrement efficace pour lutter contre les maladies respiratoires, ainsi que contre les autres maladies pour lesquelles la porte d'entrée de l'agent pathogène est mucosale.

Un autre objectif de l'invention est de proposer un tel vaccin qui soit utilisable aussi bien chez les adultes que chez les jeunes animaux.

Un objectif spécifique est de proposer un tel vaccin utilisable dans la vaccination de masse par voie mucosale des tout jeunes animaux tels que les poussins d'un jour.

Un autre objectif de l'invention est de proposer un vaccin contre l'ILT qui ait une efficacité accrue par rapport à la souche parentale et qui puisse même éventuellement permettre l'insertion et l'expression d'un gène hétérologue.

Au cours de leurs travaux sur le virus ILTV, les inventeurs ont trouvé une région génomique qui s'est révélée tout à fait appropriée comme site d'insertion de gènes hétérologues. Cela a permis de mettre au point un vaccin vivant recombinant à base d'un vecteur ILTV dans lequel est insérée au moins une séquence codant pour un immunogène aviaire, en particulier les protéines HN et F du virus de la maladie de Newcastle (NDV), et/ou la glycoprotéine gB du virus de la maladie de Marek (MDV), et/ou la protéine VP2 du virus de la maladie de Gumboro (IBDV), et/ou les protéines S et M du virus de la bronchite infectieuse (IBV). Un tel vaccin incorporant une séquence codant pour des protéines du NDV, du MDV et/ou de l'IBV assure une protection satisfaisante des animaux contre la maladie de Newcastle, contre la maladie de Marek, contre la maladie de Gumboro, et contre la bronchite infectieuse respectivement.

La présente invention a donc pour objet un vaccin vivant recombinant aviaire comprenant, comme vecteur, le virus ILTV comprenant au moins une séquence nucléotidique hétérologue, notamment codant pour, et exprimant, un polypeptide antigénique d'un agent pathogène aviaire, insérée dans le locus d'insertion formé de l'intergène situé entre les codons "stop" des COL-D et COL-E du virus ILTV et qui, dans une souche ILTV particulière, est défini entre les nucléotides 3873 et 4260 à la séquence SEQ ID NO: 1.

Si la séquence particulière décrite dans la demande (SEQ ID NO:1) provient de la souche vaccinale d'ILTV T-20 12-8-66 (vaccin LT BLEN) provenant de Select Laboratories (10026 Main Street P.O. Box 6 Berlin, Maryland 21811, USA), il est bien évident que l'homme du métier pourra utiliser les autres souches d'ILTV, compte-tenu des informations données dans la présente sur la souche vaccinale.

Le COL-E correspond au gène UL54 décrit dans l'article de M.A. Johnson *et al. (Arch. Virol*. 1995. **140**. 623-634) de la souche vaccinale australienne SA-2. La séquence nucléotidique du gène UL54 de la souche SA-2 est légèrement différente de celle de la souche T-20, ce qui entraîne des différences entre les séquences en acides aminés des gènes de ces 2 souches, et notamment à la partie C-terminale (codons STOP différents). Cet article ne suggère en aucune manière que la séquence en aval du gène UL54 puisse être utilisée comme locus d'insertion.

La séquence référencée SEQ ID NO:19 reproduit pour cette souche SA-2, une partie de la séquence équivalente à SEQ ID NO:1 (en sens inverse). L'intergène servant de locus d'insertion conformément à l'invention est en partie compris à la SEQ ID NO:19 entre Les nucléotides 2808 et 3116 (dernier nucléotide de cette séquence).

Par séquence hétérologue, on entend une séquence qui ne provient pas de ce locus d'insertion, c'est-à-dire aussi bien une séquence n'ayant pas pour origine le virus ILTV, qu'une séquence provenant d'une autre région génomique de ce virus, ou encore provenant d'une autre souche ILTV, notamment une souche virulente.

Par insertion dans la région d'insertion, on entend notamment insertion simple ou après délétion totale ou partielle du locus d'insertion.

On peut insérer une ou plusieurs cassettes d'expression chacune comprenant au moins une séquence à exprimer.

Pour exprimer la séquence insérée, on préfère utiliser un promoteur eucaryote fort tel que le promoteur CMV immédiate early (IE), le LTR du virus du sarcome de Rous (RSV), et le promoteur précoce du virus SV40.

Par promoteur CMV immédiate early (IE), on entend notamment le fragment donné dans les exemples ainsi que ses sous-fragments conservant la même activité promotrice.

Le promoteur CMV IE peut être le promoteur humain (HCMV IE) ou le promoteur murin (MCMV IE), ou encore un promoteur CMV IE d'une autre origine, par exemple du singe, du rat, du cobaye ou du porc.

D'autres promoteurs d'origine virale ou cellulaire peuvent également être utilisés. Parmi les promoteurs d'origine virale, on peut encore citer les promoteurs de gènes du virus ILTV (gènes considérés comme précoce-immédiats (ICP4, ICP27, ...), précoces (thymidine kinase, DNA helicase, ribonucléotide réductase, ...), ou tardifs (gB, gD, gC, gK, ...)), du virus de la maladie de Marek (MDV) (gènes gB, gC, pp38, pp14, ICP4, Meq,...) ou du virus de l'herpès de la dinde (herpèsvirus of turkey) (gènes gB, gC, ICP4, ...).

La séquence nucléotidique insérée dans le vecteur ILTV pour être exprimée peut être toute séquence codant pour un polypeptide antigénique, d'un agent pathogène aviaire, capable, une fois exprimé dans les conditions favorables procurées par l'invention, d'assurer une immunisation conduisant à une protection efficace de l'animal vacciné contre l'agent pathogène. On pourra donc insérer, dans les conditions de l'invention, les séquences nucléotidiques codant pour les antigènes d'intérêt pour une maladie donnée.

Cette séquence nucléotidique insérée dans le vecteur ILTV peut également coder pour un polypeptide immunomodulateur, et notamment une cytokine.

De manière remarquable, les vaccins selon l'invention pourront être utilisés pour la vaccination *in ovo*, des poussins d'un jour ou plus et des adultes. Différentes voies d'administration pourront être utilisées: la voie parentérale, ou les voies mucosales telles que oronasale (eau de boisson, aérosol), conjonctivale (goutte dans l'oeil) ou cloacale, avec une préférence pour les voies permettant une vaccination mucosale de masse (eau de boisson, aérosol).

L'invention se révèle particulièrement utile aussi bien pour la protection contre les pathologies respiratoires que contre les pathologies systémiques en bloquant les voies d'entrée naturelles de l'agent pathogène.

L'invention peut notamment être utilisée pour l'insertion d'une séquence nucléotidique codant convenablement pour une protéine antigénique du virus NDV et en particulier, la glycoprotéine HN ou la glycoprotéine F. On obtient ainsi un vaccin vivant recombinant assurant, en plus d'une protection contre la laryngotrachéite infectieuse, une protection satisfaisante contre la maladie de Newcastle.

Le vaccin recombinant contre la maladie de Newcastle contiendra de préférence de 10 à 10⁴ PFU/dose.

D'autres cas préférés de l'invention sont l'insertion de séquences nucléotidiques codant pour des antigènes d'autres agents pathogènes aviaires, et notamment, mais de manière non limitative, des antigènes du virus de la maladie de Marek, en particulier gènes gB, gC, gD, et gH+gL (WO-A-90/02803), du virus de la maladie de Gumboro, en particulier gène VP2, du virus de la bronchite infectieuse (IBV), en particulier gènes S et M (M. Binns *et al*., *J*. *Gen. Virol*. 1985. **66.** 719-726 ; M. Boursnell *et al. , Virus Research* 1984. **1**. 303-313), du virus de l'anémie du poulet (CAV), en particulier VP1 (52 kDa) + VP2 (24 kDa) (N.H.M. Noteborn *et al*., *J*. *Virol*. 1991. **65.** 3131-3139), du virus ILTV, en particulier les gènes codant pour gB (A.M. Griffin, *J*. *Gen*. *Virol*. 1991. **72**. 393-398), ou pour gD (M.A. Johnson *et al*., *DNA Sequence- The Journal of Sequencing and Mapping* 1995. **Vol. 5**. pp191-194. Harwood Academic Publishers GmbH), ou pour gp60 (K.K. Kongsuwan *et al., Virus Genes* 1993. **7**. 297-303), et du virus du syndrome infectieux du gonflement de la tête ("swollen head syndrome" ou pneumovirose du poulet ou turkey rhinotracheitis virus (TRTV) de la dinde; pneumovirus), en particulier la glycoprotéine de fusion F (Q. Yu *et al*., *J. Gen. Virol*. 1991. **72**. 75-81), ou la glycoprotéine d'attachement G (R. Ling *et al., J*. *Gen. Virol*. 1992. **73**. 1709-1715; K. Juhasz et J. Easton, *J. Gen. Virol*. 1994. **75**. 2873-2880). Les doses seront de préférence les mêmes que celles pour le vaccin de Newcastle.

Dans le cadre de la présente invention, on peut bien entendu insérer plus d'une séquence hétérologue dans le même virus ILTV, notamment dans ce locus. On peut notamment y insérer des séquences provenant d'un même virus ou de virus différents, ce qui comprend également l'insertion de séquences d'ILTV et d'un autre virus aviaire. On peut également y associer des séquences codant pour des immunomodulateurs, et en particulier des cytokines.

Par exemple, on associe au promoteur CMV IE un autre promoteur de façon que leurs extrémités 5' soient adjacentes (ce qui implique des transcriptions dans des sens opposés), ce qui permet d'insérer, dans la zone d'insertion, deux séquences nucléotidiques, l'une sous la dépendance du promoteur CMV IE, l'autre sous celle du promoteur associé. Cette construction est remarquable par le fait que la présence du promoteur CMV IE, et notamment de sa partie activatrice (enhancer), active la transcription induite par le promoteur associé. Le promoteur associé peut être en particulier un promoteur d'un gène du virus ILTV ou du virus MDV ou HVT.

Un cas intéressant de l'invention est un vaccin comprenant une séquence nucléotidique codant pour HN de NDV et une séquence nucléotidique codant pour F de NDV ou un antigène d'une autre maladie aviaire, notamment celles citées plus haut, l'un des gènes étant sous le contrôle du promoteur CMV IE, et l'autre sous le contrôle du promoteur associé.

On peut aussi monter deux promoteurs CMV IE d'origines différentes avec leurs extrémités 5' adjacentes.

L'expression de plusieurs gènes hétérologues insérés dans le locus d'insertion peut également être rendu possible par insertion entre les cadres ouverts de lecture de ces gènes d'une séquence appelée "IRES" (Internal Ribosome Entry Site) provenant notamment d'un picomavirus tel le virus de la maladie vésiculaire du porc (swine vesicular disease virus, SVDV; B. - F. Chen *et al*., *J. Virology*, 1993, **67**, 2142-2148), le virus de l'encéphalomyocardite (EMCV; R.J. Kaufman *et al*., *Nucleic Acids Research,* 1991, **19**, 4485-4490), le virus de la fièvre aphteuse (FMDV; N. Luz et E. Beck, *J. Virology*, 1991, **65,** 6486-6494), ou encore d'une autre origine. Le contenu des 3 articles cités est incorporé par référence. La cassette d'expression de deux gènes aurait donc la structure minimale suivante: promoteur - gène 1 - IRES - gène2 - signal de polyadénylation. Le vaccin vivant recombinant selon l'invention pourra donc comprendre, insérée dans le locus d'insertion, une cassette d'expression comprenant successivement un promoteur, deux ou plusieurs gènes séparés deux à deux par un IRES, et un signal de polyadénylation.

En plus de l'insertion dans le locus selon l'invention, on peut réaliser une ou plusieurs autres insertions, une ou plusieurs mutations, ou une ou plusieurs délétions ailleurs dans le génome; si la souche parentale est virulente, on peut par exemple inactiver (par délétion, insertion ou mutation) des gènes impliqués dans la virulence tels que le gène thymidine kinase, le gène ribonucléotide réductase, le gène gE,... Dans tous les cas, l'insertion dans un autre locus que celui décrit dans l'invention, permet d'exprimer d'autres gènes.

La présente invention a aussi pour objet un vaccin contre l'ILT comprenant un virus ILTV recombinant dans lequel on a inséré en amont des gènes codant pour des immunogènes majeurs de l'ILTV, de préférence les gènes codant pour gB (A.M. Griffin, *J. Gen. Virol*. 1991. **72.** 393-398), ou pour gD (M.A. Johnson *et al., DNA Sequence-The Journal of Sequencing and Mapping* 1995. **Vol. 5**. pp191-194. Harwood Academic Publishers GmbH), ou pour gp60 (K.K. Kongsuwan *et al., Virus Genes* 1993. **7**. 297-303), un promoteur exogène, en particulier un promoteur fort tel que décrit plus haut. Cela permet d'augmenter le niveau d'expression de l'un ou plusieurs de ces gènes et ainsi conduire à un vaccin à efficacité accrue contre l'ILT. On peut bien sûr combiner cela avec une construction telle que décrite plus haut comprenant l'insertion d'une séquence hétérologue dans le locus d'insertion.

La présente invention a aussi pour objet une formule de vaccin multivalent, comprenant, en mélange ou à mélanger, un vaccin tel que défini plus haut avec un autre vaccin, et notamment un autre vaccin vivant recombinant aviaire tel que défini plus haut, ces vaccins comprenant des séquences insérées différentes, notamment de pathogènes différents.

La présente invention a aussi pour objet une méthode de préparation des vaccins selon l'invention, telle qu'elle ressort de la description.

La présente invention a aussi pour objet une méthode de vaccination aviaire comprenant l'administration d'un vaccin vivant recombinant ou d'une formule de vaccin multivalent tel que défini plus haut. Elle a notamment pour objet une telle méthode pour la vaccination *in ovo*, des poussins d'un jour ou plus et des adultes. Différentes voies d'administration du vaccin peuvent être utilisées (voir plus haut) avec une préférence pour les voies permettant une vaccination de masse par voie mucosale (aérosol, eau de boisson), la dose de vaccin étant choisie de préférence entre 10¹ et 10⁴ par animal.

La présente invention a aussi pour objet un virus ILTV comprenant au moins une séquence nucléotidique hétérologue telle que décrite ci-dessus insérée dans le locus d'insertion tel que défini plus haut.

La présente invention a encore pour objet tout ou partie de la séquence SEQ ID NO: 1. Par partie de la séquence, on entend non seulement les COLs caractérisés pris séparément et leurs fragments, mais aussi la région intergénique située entre les COLs D et E, et les fragments situés de chaque côté de cette région intergénique, qui peuvent, le cas échéant inclure une partie de cette région intergénique, ces fragments pouvant servir de bras flanquant pour une recombinaison homologue, technique parfaitement connue de l'homme du métier. De manière générale, mais sans que cela soit limitatif, les bras flanquants pourront avoir de 100 à 1500 paires de bases.

L'invention va être maintenant décrite plus en détail à l'aide d'exemples de réalisation non limitatifs, pris en référence au dessin, dans lequel :
- **Figure 1 :**: Carte de restriction du fragment cloné et position des COLs
- **Figure 2 :**: Séquence de 7082 pb et traduction des COLs A, B, C, D, E et F de la souche vaccinale T-20 de Select Laboratories
- **Figure 3 :**: Schéma d'obtention du plasmide pEL168
- **Figure 4 :**: Schéma d'obtention du plasmide pEL024
- **Figure 5 :**: Schéma d'obtention du plasmide pEL027
- **Figure 6 :**: Schéma du plasmide pEL169
- **Figure 7 :**: Schéma d'obtention du plasmide pCD009
- **Figure 8 :**: Schéma d'obtention du plasmide pEL070
- **Figure 9 :**: Schéma du plasmide pEL170
- **Figure 10 :**: séquence du gène HN du NDV
- **Figure 11 :**: Schéma d'obtention du plasmide pEL030
- **Figure 12 :**: Schéma du plasmide pEL171
- **Figure 13 :**: Schéma du plasmide pEL033
- **Figure 14 :**: Schéma du plasmide pEL172
- **Figure 15 :**: Schéma de double cassette d'expression
- **Figure 16 :**: Schéma d'obtention du plasmide pCD011
- **Figure 17 :**: Schéma du plasmide pEL181
- **Figure 18 :**: Séquence de 3116 pb et traduction des UL53 et 54 de la souche SA-2

### Liste des séquences :

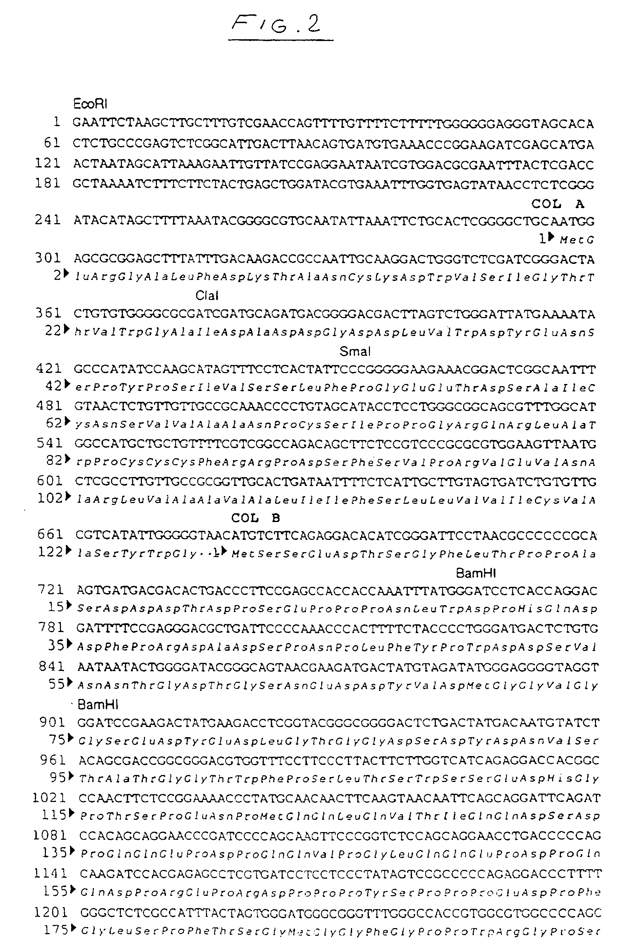

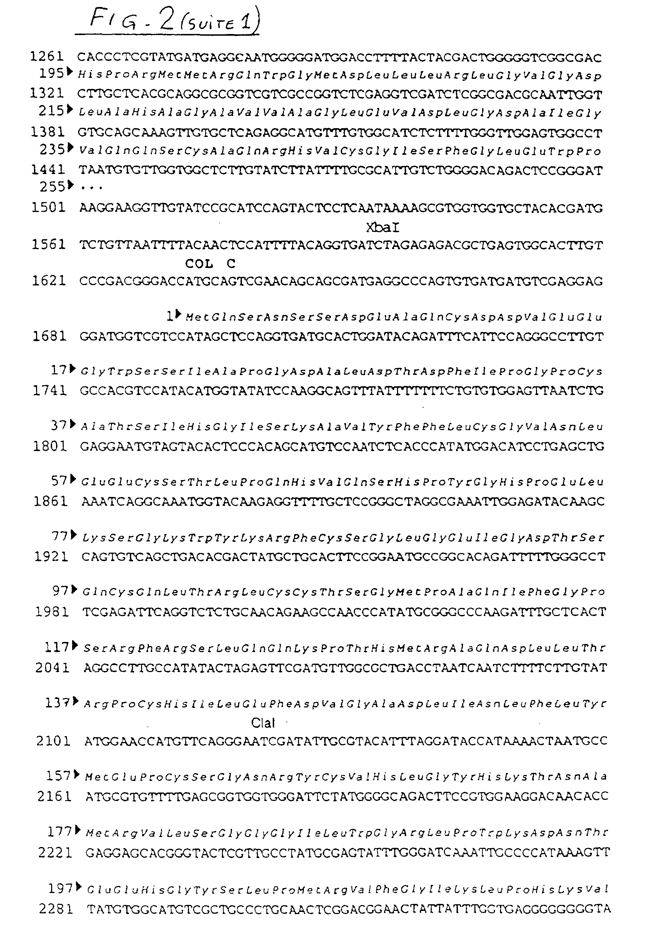

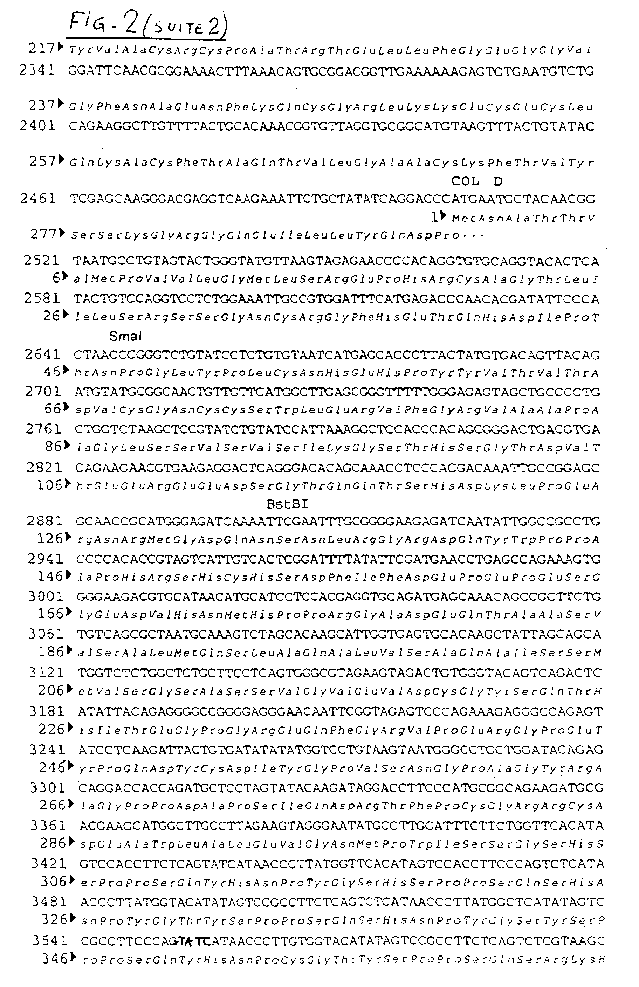

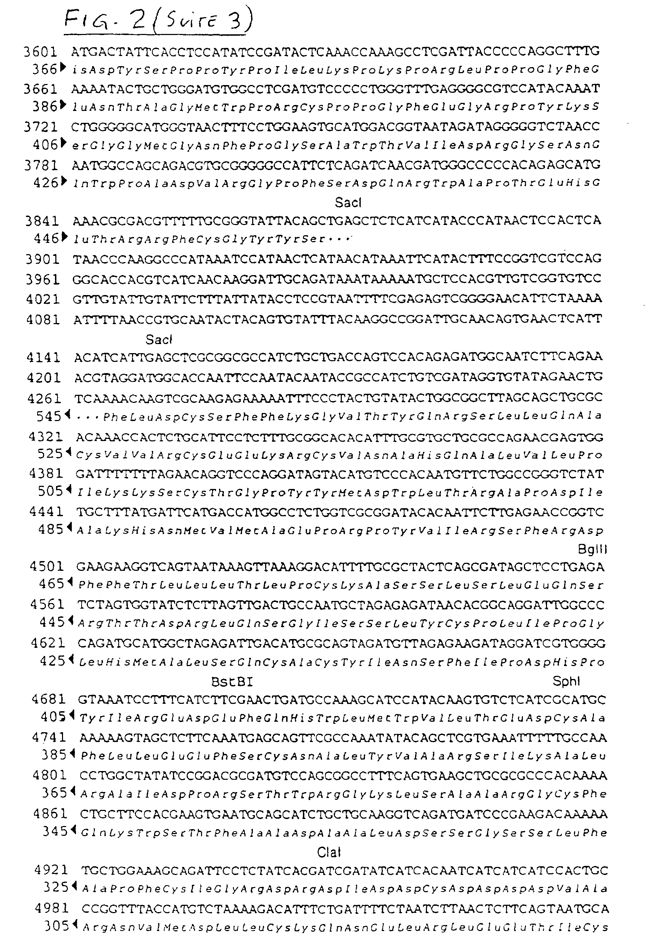

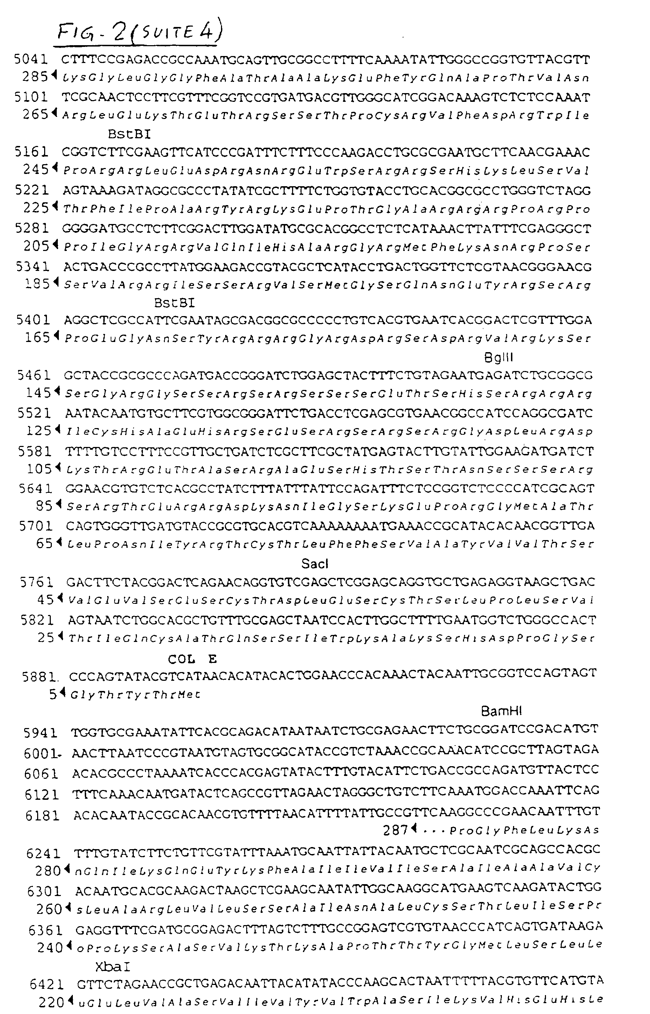

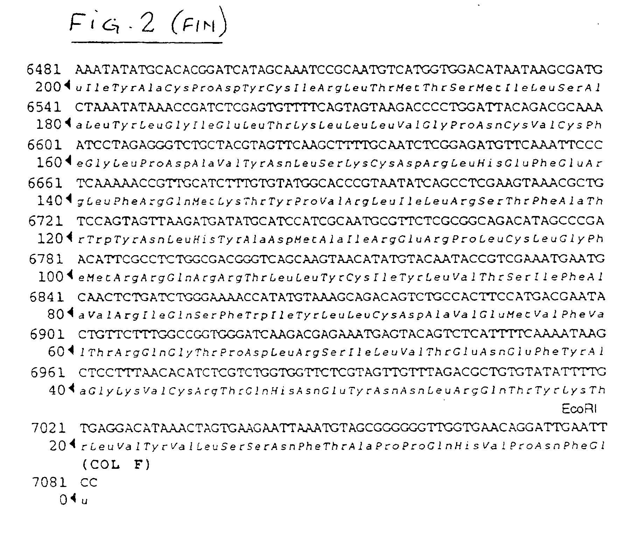

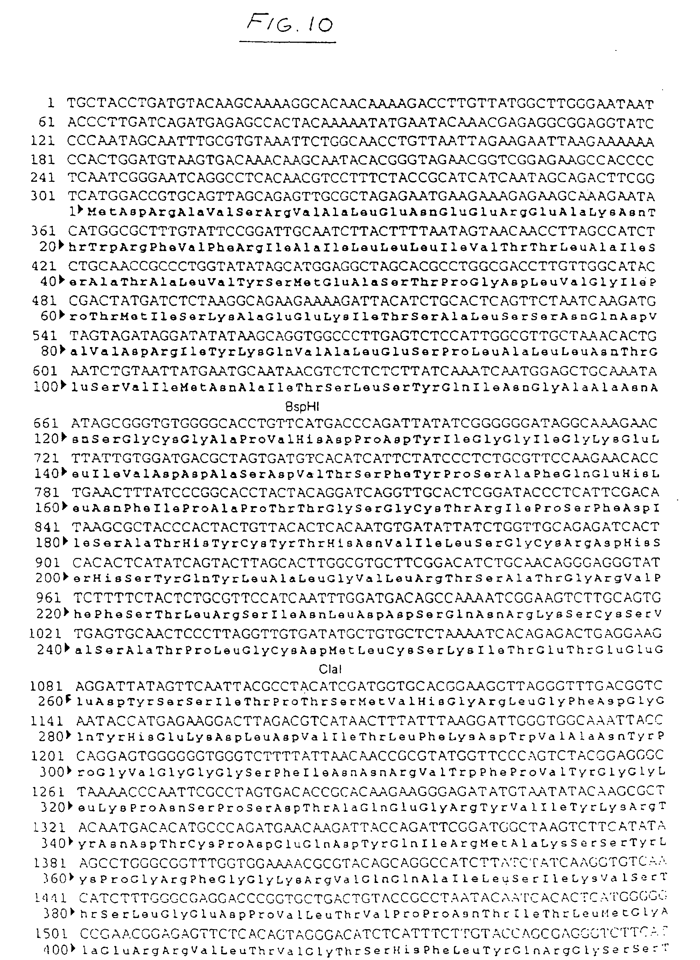

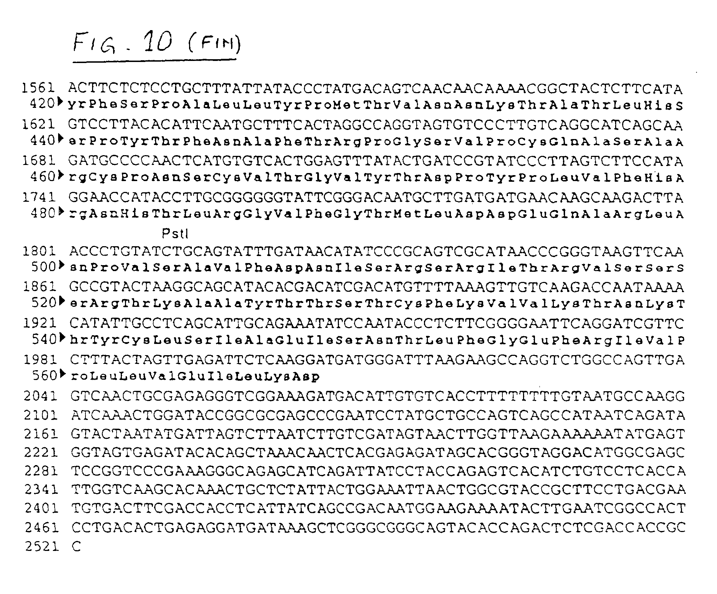

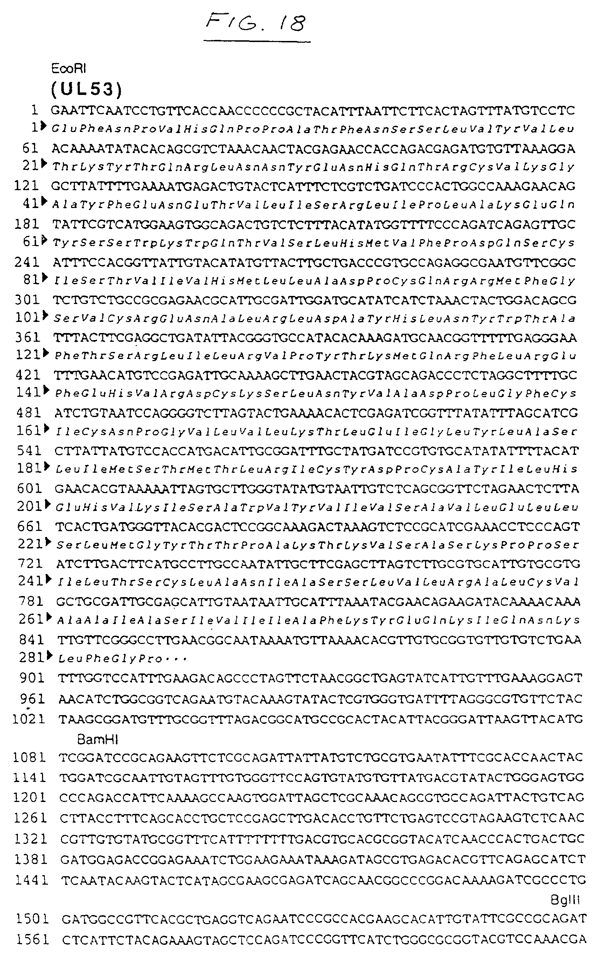

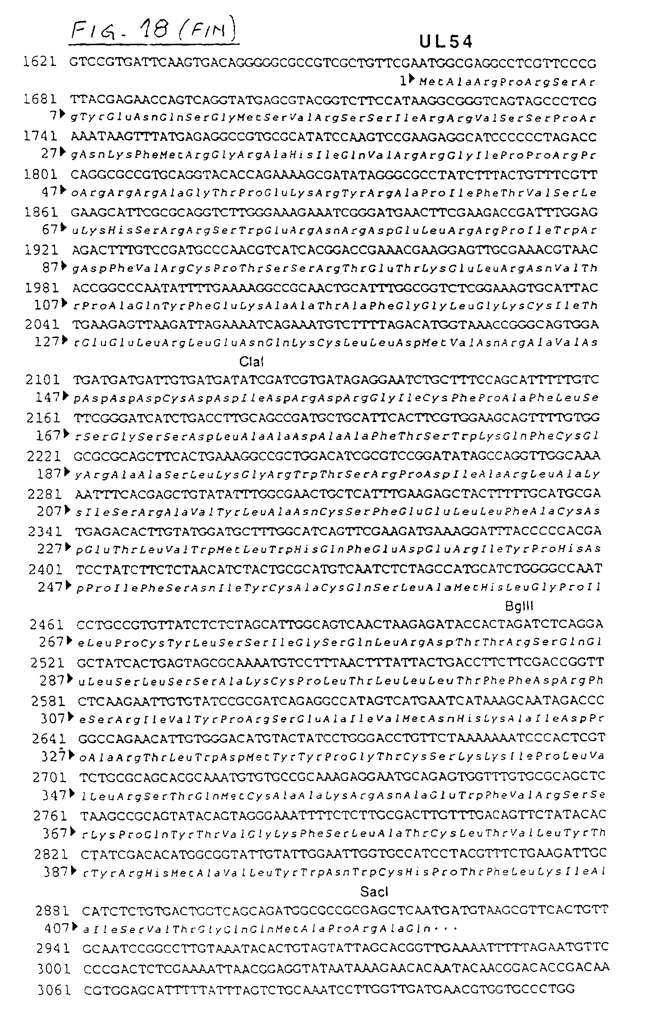

- **SEQ ID NO:1**: Séquence du fragment *Eco*RI*-Eco*RI (7082 pb, voir figure 2)
- **SEQ ID NO:2**: Oligonucléotide EL005
- **SEQ ID NO:3**: Oligonucléotide EL006
- **SEQ ID NO:4**: Oligonucléotide EL007
- **SEQ ID NO:5**: Oligonucléotide EL008
- **SEQ ID NO:6**: Oligonucléotide MB070
- **SEQ ID NO:7**: Oligonucléotide MB071
- **SEQ ID NO:8**: Séquence du gène HN du NDV (voir figure 10)
- **SEQ ID NO:9**: Oligonucléotide EL071
- **SEQ ID NO:10**: Oligonucléotide EL073
- **SEQ ID NO:11**: Oligonucléotide EL074
- **SEQ ID NO:12**: Oligonucléotide EL075
- **SEQ ID NO:13**: Oligonucléotide EL076
- **SEQ ID NO:14**: Oligonucléotide EL077
- **SEQ ID NO:15**: Oligonucléotide CD001
- **SEQ ID NO:16**: Oligonucléotide CD002
- **SEQ ID NO:17**: Oligonucléotide CD003
- **SEQ ID NO:18**: Oligonucléotide CD004
- **SEQ ID NO:19**: Séquence de la souche SA-2 (3116 bp, à partir du site *Eco*RI en position 1696 de la séquence de Genbank n° d'accès L34065 ; voir figure 18)

### EXEMPLES

Toutes les constructions de plasmides ont été réalisées en utilisant les techniques standards de biologie moléculaire décrites par Sambrook J. *et al*. (*Molecular Cloning: A Laboratory Manual.* 2^{nd} Edition. Cold Spring Harbor Laboratory. Cold Spring Harbor. New York. 1989). Tous les fragments de restriction utilisés pour la présente invention ont été isolés en utilisant le kit "Geneclean" (BI0101 Inc. La Jolla, CA).

Le virus utilisé comme virus parental peut être choisi parmi les souches vaccinales décrites dans J.R. Andreasen *et al*. (*Avian Diseases* 1990. **34**. 646-656) ou la souche T-20 12-8-66 vaccin LT BLEN provenant de Select laboratories 10026 Main Street P.O. Box 6 Berlin, Maryland 21811, USA. On peut également utiliser des souches virulentes telles que la souche de Lütticken (voir ci-dessus), la souche N-71851 (ATCC VR-783) ou la souche 83-2 de l'USDA, que l'on peut atténuer par les techniques connues, par exemple celle décrite dans WO-A-95/08622.

### Exemple 1: Culture du virus ILTV:

Le virus ILTV (souche T20 de Select Laboratories) est cultivé sur des cellules primaires de reins de poulets (CRP); ces cellules sont mises en culture en milieu MEM complémenté avec 3% de sérum de veau foetal (SVF) dans des flacons de culture de 75 cm² (2 10⁵ cellules/cm²) un ou deux jours avant inoculation.

Le jour de l'inoculation, un flacon de 1000 doses de vaccin lyophilisé est resuspendu dans 10 ml de milieu MEM complémenté avec 1% de SVF; environ 0,5 ml de cette solution est ensuite déposé sur la culture de CRP. Le lendemain, le milieu est changé, et le surlendemain, lorsque l'effet cytopathogène (ECP) se généralise, les flacons de culture sont congelés à -70°C.

La culture du virus ILTV peut également être faite sur des cellules immortalisées de foie de poulet, et notamment sur la lignée LMH (W.M. Schnitzlein *et al*., *Avian Diseases* 1994.**38**.211-217).

### Exemple 2: Préparation de l'ADN génomique de l'ILTV:

Après 2 cycles de congelation/décongelation, la culture d'ILTV (2 flacons de 75 cm²) est récoltée et centrifugée à basse vitesse (5000 tr/min dans un rotor 20, centrifugeuse Beckman JA21, pendant 5 minutes) pour éliminer les gros débris cellulaires. Le surnageant est ensuite ultracentrifugé (100000 tr/min rotor TLA100.3, centrifugeuse Beckman TL100, pendant 1 heure). Le culot est alors repris dans 1,6 ml de TEN-SDS (Tris pH 8,0 10mM; EDTA 1mM; NaCl 0,5M; sodium dodecyl sulfate 0,5%), et 35 µl d'une solution de protéinase K à 20 mg/ml sont ensuite ajoutés; la solution est incubée 3 à 4 heures au bain marie à 37°C, et l'ADN est ensuite extrait 3 fois au phénol/chloroforme et 1 fois au chloroforme, puis il est précipité à l'éthanol à -20°C. Après centrifugation, le culot est rincé à l'éthanol 70%, séché et resuspendu dans 200 µl TE (Tris pH8.0 10mM; EDTA 1mM). La concentration en acide nucléique est ensuite dosée au spectrophotomètre (DO₂₆₀). L'ADN peut être directement digéré par les enzymes de restriction appropriées, pour être ensuite cloné dans le plasmide pBlue Script II SK⁺ ; de même, il pourra également être utilisé dans les expériences de transfection pour l'obtention d'un virus recombinant.

### Exemple 3: Isolement et purification de virus recombinant ILTV

Le plasmide donneur composé d'une cassette d'expression d'un polypeptide inséré entre deux régions flanquantes du locus d'insertion est digéré par une enzyme de restriction permettant la linéarisation du plasmide, puis il est extrait avec un mélange phénol/chloroforme, précipité avec de l'éthanol absolu, et repris dans de l'eau stérile. Des cellules CRP primaires de 24 heures sont ensuite transfectées avec le mélange suivant: 0,2 à 1 µg de plasmide donneur linéarisé + 2 à 5 µg d'ADN viral d'ILTV (préparé comme dans l'exemple 2) dans 300 µl de milieu OptiMEM (Gibco BRL Cat# 041-01985H) et 100 µg de LipofectAMINE dilués dans 300 µl de milieu (volume final du mélange = 600 µl). Ces 600µl sont ensuite dilués dans 3 ml (volume final) de milieu et étalés sur 5.10⁶ CRP. Le mélange est laissé en contact avec les cellules pendant 5 heures, puis éliminé et remplacé par 5 ml de milieu de culture. Les cellules sont alors laissées en culture pendant 3 à 8 jours à + 37°C, puis, lorsque l'effet cytopathogène est apparu, elles sont congelées à -70°C. Après décongelation et éventuellement sonication, cette population virale est clonée en dilution limite en microplaques (96 puits) afin d'isoler une population homogène de virus recombinant. Ces plaques sont laissées en culture pendant 1 à 3 jours, puis le surnageant est récolté dans une plaque 96 puits vide et la plaque contenant les surnageants est placée à 4°C ou à -70°C. Les cellules restant dans les autres plaques sont ensuite fixées à l'acétone 95% pendant 20 à 30 minutes à - 20°C, ou pendant 5 minutes à température ambiante. Une réaction d'immunofluorescence indirecte (IFI) est réalisée avec un anticorps monoclonal dirigé contre le polypeptide exprimé pour rechercher les plages exprimant ce polypeptide. Un nouveau clonage est ensuite effectué de la même manière (en dilution limite en plaques 96 puits) à partir du surnageant présent dans les cupules des plaques mises à 4°C ou à -70°C et correspondant aux cupules présentant des plages positives en IFI. En général, 4 cycles d'isolement successifs (dilution limite, récolte du surnageant, contrôle des cellules par IFI, dilution limite à partir du surnageant...) suffisent pour obtenir des virus recombinants dont la totalité de la progénie présente une fluorescence spécifique. L' ADN génomique de ces virus recombinants est caractérisé au niveau moléculaire par des techniques classiques de PCR et de Southem blot en utilisant les oligonucléotides et les sondes d'ADN appropriés.

L'isolement de virus recombinant peut également se faire par hybridation avec une sonde spécifique de la cassette d'expression insérée. Pour cela, la population virale récoltée après transfection est diluée et déposée sur des cellules CRP (cultivées en boîte de Petri) de manière à obtenir des plages isolées. Après un contact d'1 heure à 37°C, le milieu d'infection est éliminé et remplacé par 5 ml de milieu MEM à 1 % d'agarose, maintenu en surfusion à 42°C. Lorsque l'agarose est solidifié, les boîtes sont incubées 48 à 72 heures à 37°C en étuve CO₂ jusqu'à apparition de plages. La couche d'agarose est alors éliminée et un transfert des plages virales est réalisé sur une membrane stérile de nitrocellulose de même diamètre que la boîte de Petri ayant servi à la culture. Cette membrane est elle-même transférée sur une autre membrane de nitrocellulose de manière à obtenir une "copie" inversée du premier transfert. Les plages transférées sur cette dernière copie sont alors hybridées, selon les techniques usuelles connues-de l'homme de l'art, avec un fragment d'ADN de la cassette d'expression marqué à la digoxigénine (DNA Labelling Kit, Boehringer Mannheim, CAT # 1175033). Après hybridation, lavages et mise en contact avec le substrat de révélation, la membrane de nitrocellulose est mise en contact avec un film autoradiographique. Les images d'hybridation positive sur cette membrane indiquent quelles sont les plages qui contiennent des virus ILTV recombinants ayant inséré la cassette d'expression. Les plages correspondant à ces plages positives sont découpées stérilement sur la première membrane de nitrocellulose, placées dans un tube Eppendorf contenant 0,5 ml de milieu MEM et soniquées pour libérer les virions de la membrane. Le milieu contenu dans le tube Eppendorf est ensuite dilué en milieu MEM et les dilutions ainsi obtenues servent à infecter de nouvelles cultures de cellules CRP.

### Exemple 4: Clonage et caractérisation d'une région génomique de l'ILTV

L'ADN extrait du virus ILTV a été digéré par l'enzyme de restriction *Eco*RI pendant 2 heures à 37°C. L' enzyme de restriction a ensuite été éliminé par une extraction au phénol/chloroforme, suivie d'une précipitation à l'éthanol. Les fragments résultant de cette digestion ont ensuite été ligaturés (une nuit à 14°C) avec le plasmide pBlueScriptII SK+ (pBS SK+; Stratagene) digéré par *Eco*RI et traité à la phosphatase alcaline; l'analyse des clones obtenus après transformation de bactéries *E. coli* DH5α et culture sur boîtes de milieu complémenté en ampiciline a permis d'identifier des inserts *Eco*RI*-Eco*-RI de tailles différentes, dont un fragment d'environ 7 kb (plasmide pEL133).

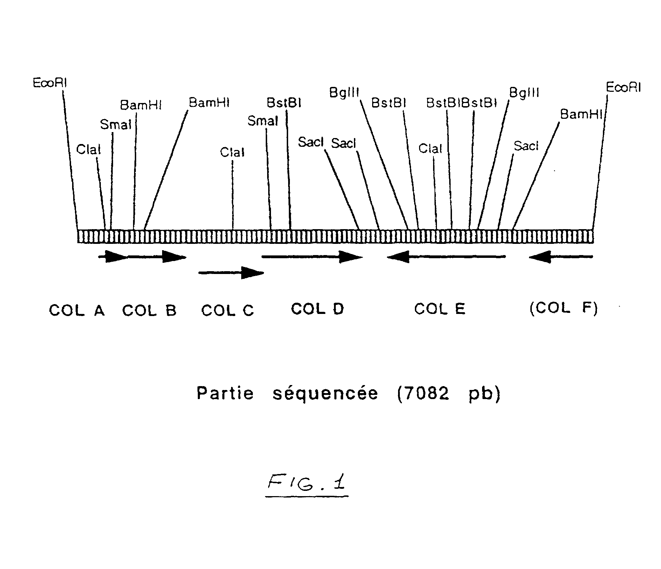

Le séquençage complet de l'insert présent dans pEL133 (voir figure 1) a permis de mettre en évidence cinq cadres ouverts de lecture (COLs) complets d'au moins 120 acides aminés (COLs A, B, C, D et E), et une partie d'un autre COL (COL F). La carte de restriction de cette région génomique clonée et séquencée, est montrée à la figure 1; la séquence de 7082 pb (SEQ ID NO:1) est montrée à la figure 2. La position et la séquence en acides aminés des COLs A, B, C, D, E et F sont également montrées sur les figures 1 et 2 respectivement.
La séquence entre les codons STOP des COLs D et E (position de 3873 à 4260 sur SEQ ID NO:1), est utilisable pour insérer des cassettes d'expression de polypeptides dans le génome de l'ILTV. Cette séquence est appelée locus d'insertion. L'insertion peut se faire avec ou sans délétion dans la région intergénique (voir exemple 5).

### Exemple 5: Construction du plasmide donneur pEL168 pour l'insertion dans la région intergénique entre les COLs D et E

Le plasmide pBluescript II SK+ (pPB SK+ ; Stratagene) a été digéré par les enzymes *Xho*I et *Hind*II, et ensuite traité à l'ADN polymérase (fragment de Klenow) en présence de dNTP pour rendre les bouts francs. Après ligature et transformation des bactéries *E. coli,* le clone pEL166 (2957 pb) a été obtenu.

Le plasmide pEL166 a été digéré par les enzymes *Sca*II et *Xba*I, et ensuite traité à l'ADN polymérase (fragment de Klenow) en présence de dNTP pour rendre les bouts francs. Après ligature et transformation des bactéries *E*. *coli,* le clone pEL167 (2944 pb) a été obtenu.

Les oligonucléotides EL005 (SEQ ID No:2) et EL006 (SEQ ID No:3) ont servi d'amorce pour une première amplification en chaîne par la Taq polymérase (PCR). Les oligonucléotides EL007 (SEQ ID No:4) et EL008 (SEQ ID No:5) ont servi d'amorce pour une deuxième amplification en chaîne par la Taq polymérase (PCR).

Les PCR ont été effectuées en présence de tampon PCR, de dNTP, d'ADN du plasmide pEL133, de Taq polymérase, et pour la première PCR, des oligonucléotides EL005 et EL006, et pour la deuxième PCR, des oligonucléotides EL007 et EL008.

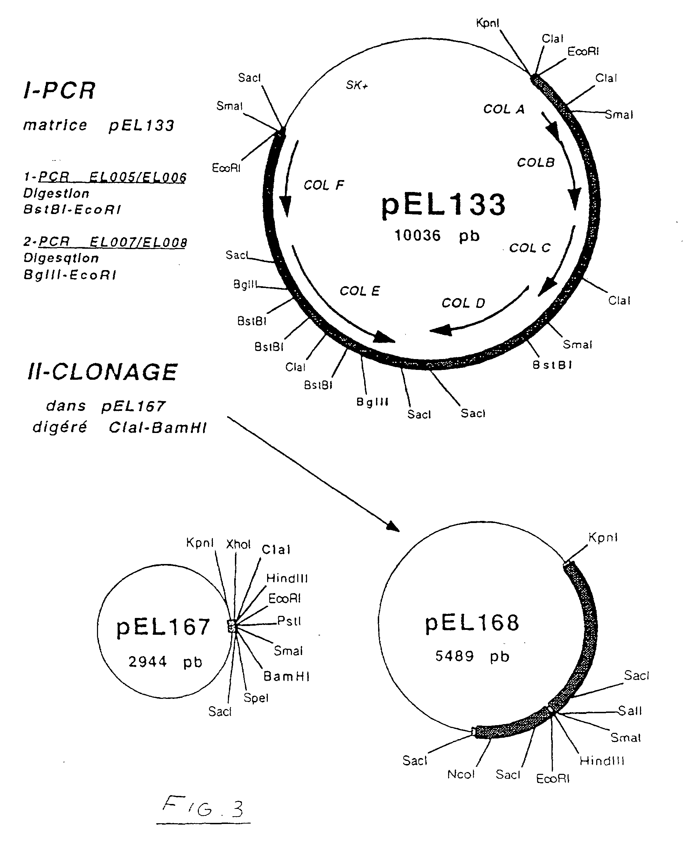

Pour les deux PCR, 25 cycles ont été effectués (30 secondes à 94°C ; 30 secondes à 60°C et 30 secondes à 72°C). Les produits des deux PCR ont été purifiés par une extraction au phénol/chloroforme, suivie d'une purification par l'éthanol. Le produit de la première PCR (EL005/EL006) a ensuite été digéré par les enzymes de restriction *Bst*BI et *Eco*RI pendant 2 h à 37°C pour donner un fragement d'ADN *Bst*BI*-Eco*RI de 1132 pb qui a été élué après électrophorèse en gel d'agarose. Le produit de la deuxième PCR (EL007/EL008) a ensuite été digéré par les enzymes de restriction *Bgl*II et *Eco*RI pendant 2 h à 37°C pour donner un fragment d'ADN *Bgl*II*-Eco*RI de 550 pb qui a été élué après électrophorèse en gel d'agarose. Le plasmide pEL167 a été digéré par les enzymes *Cla*I et *Bam*HI. Les deux fragements de PCR *Bst*BI-*Eco*RI (1132 pb) et *Bgl*II*-Eco*RI (550 pb) ont été ligaturés une nuit à 14°C avec le plasmide pEL167 digéré par *Cla*I et *Bam*HI. Après transformation des bactéries *E. coli,* et culture sur boîtes de milieu complémenté en ampicilline, le clone pEL168 (4589 pb), comprenant un polylinker *Eco*RI *- Hind*III - *Sma*I - *Sal*I a été obtenu (voir schéma d'obtention de pEL168 à la figure 3).

### Exemple 6: Construction du plasmide donneur pEL169 pour l'insertion d'une cassette d'expression du gène VP2 de l'IBDV sous controle du promoteur HCMV IE dans le site intergénique entre les COLs D et E, et isolement de vILTV13:

### 6.1 - Clonage du gène VP2 du virus de la maladie de Gumboro (IBDV) et construction d'une cassette d'expression de VP2 sous controle du promoteur HCMV IE

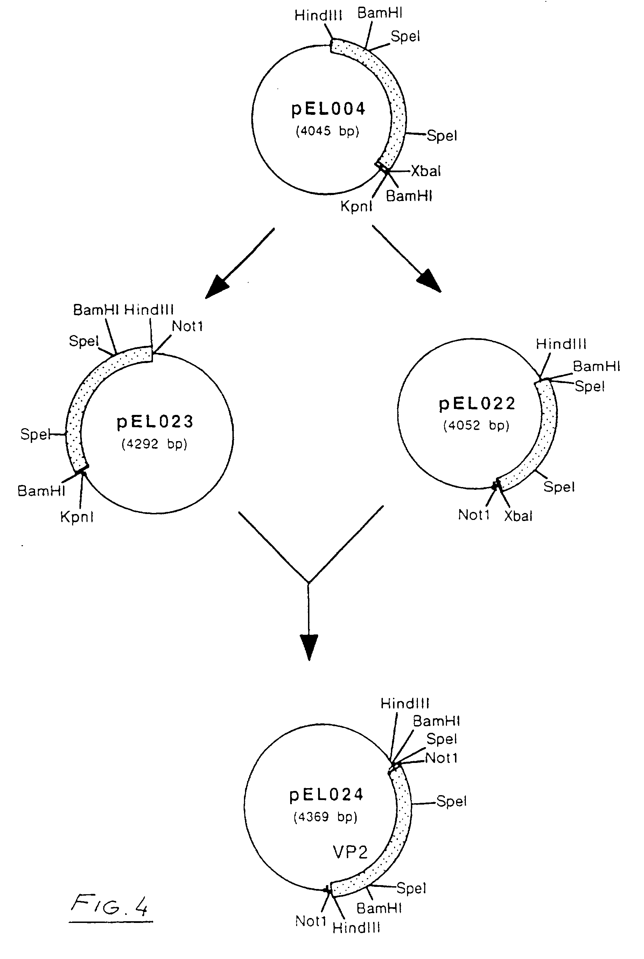

Le plasmide pEL004 (voir figure 4; = plasmide pGH004 décrit dans la demande de brevet français 92.13109) contenant le gène IBDV VP2 sous forme d'une cassette *Bam*HI-*Hind*III a été digéré par *Bam*HI et *Xba*I pour isoler le fragment *Bam*HI-*Xba*I (gène VP2 tronqué) de 1104 pb. Ce fragment a été cloné dans le vecteur pBS SK+, préalablement digéré avec *Xba*I et *Bam*HI pour donner le plasmide pEL022 de 4052 pb (figure 4). Le vecteur pBS-SK+ a été digéré par *Eco*RV et *Xba*I, puis ligaturé sur lui-même pour donner pBS-SK* (modifié). Le plasmide pEL004 a été digéré par *Kpn*I et *Hind*III pour isoler le fragment *Kpn*I*-Hind*III de 1387 pb contenant le gène IBDV VP2 complet. Ce fragment a été cloné dans le vecteur pBS-SK*, préalablement digéré par *Kpn*I et *Hind*III, pour donner le plasmide pEL023 de 4292 pb (figure 4). Le plasmide pEL022 a été digéré par *Bam*HI et *Not*I pour isoler le fragment *Bam*HI*-Not*I de 1122 pb (fragment A). Le plasmide pEL023 a été digéré par *Bam*HI et *Not*I pour isoler le fragment *Bam*HI*-Not*I de 333 pb (fragment B). Les fragments A et B ont été ligaturés ensemble avec le vecteur pBS-SK+, préalablement digéré par *Not*I et traité avec la phosphatase alcaline, pour donner le plasmide pEL024 de 4369 pb (figure 4).

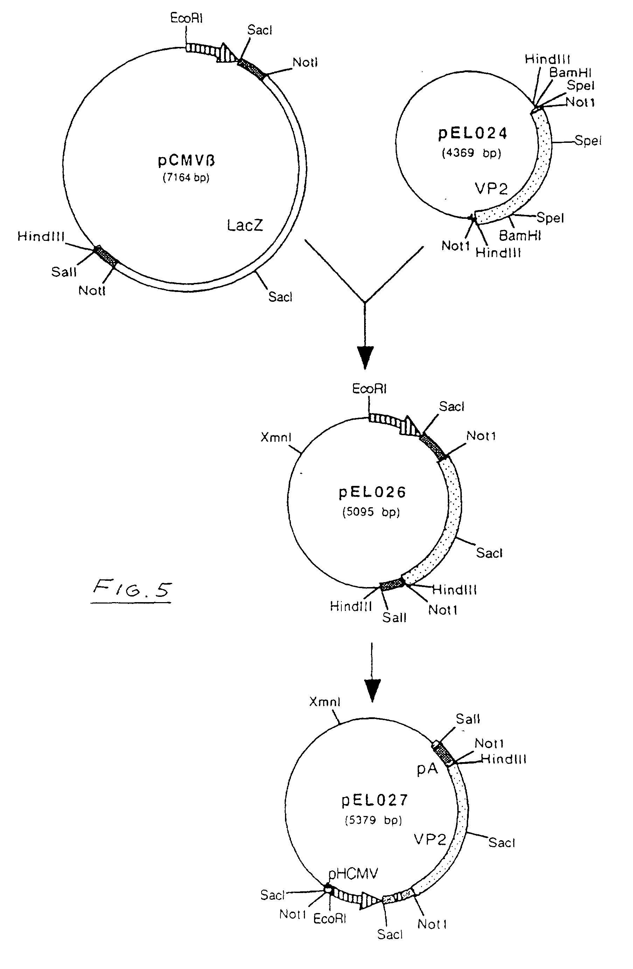

Le plasmide pEL024 a été digéré par *Not*I pour isoler le fragment *Not*I*-Not*I de 1445 pb. Ce fragment a été ligaturé avec le plasmide pCMVß (Clontech Cat# 6177-1, figure 5), préalablement digéré par *Not*I, pour donner le plasmide pEL026 de 5095 pb (figure 5).

Le plasmide pEL026 a été digéré par *EcoR*I, *Sal*I et *Xmn*I pour isoler le fragment *Eco*RI*-Sal*I de 2428 pb. Ce fragment a été ligaturé avec le vecteur pBS-SK+, préalablement digéré par *Eco*RI et *Sal*I, pour donner le plasmide pEL027 de 5379 pb (Figure 5).

### 6.2 - Construction du plasmide donneur pEL169

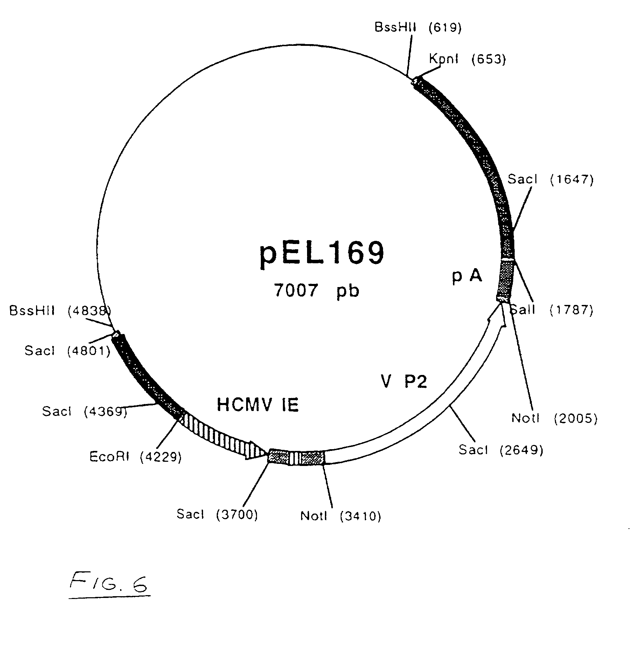

Le plasmide pEL027 a été digéré par *Eco*RI, *Sal*I et *Xmn*I pour isoler le fragment *Eco*RI *Sal*I de 2428 pb. Ce fragment a été ligaturé dans le plasmide pEL168 (voir exemple 5 et figure 3), préalablement digéré par *Eco*RI et *Sal*I, pour donner le plasmide pEL169 de 7007 pb (figure 6).

### 6.3 - Isolement et purification du virus recombinant vILTV13

Le virus vILTV13 a été isolé et purifié après cotransfection de l'ADN du plasmide pEL169 préalablement linéarisé par l'enzyme *Kpn*I et de l'ADN viral, comme décrit dans l'exemple 3. Ce recombinant contient une cassette HCMV-IE/IBDV VP2 dans le site intergénique entre les COLs D et E du virus ILTV (voir exemple 5).

### Exemple 7: Construction du plasmide donneur pEL170 pour l'insertion d'une cassette d'expression du gène VP2 de l'IBDV sous controle du promoteur MCMV IE dans le site intergénique entre les COLs D et E et isolement dé vILTV14:

### 7.1 - Construction de pEL070 contenant une cassette d'expression du gène VP2 de l'IBDV sous controle du promoteur immediate early (IE) du MCMV (Mouse CyoMegalo Virus)

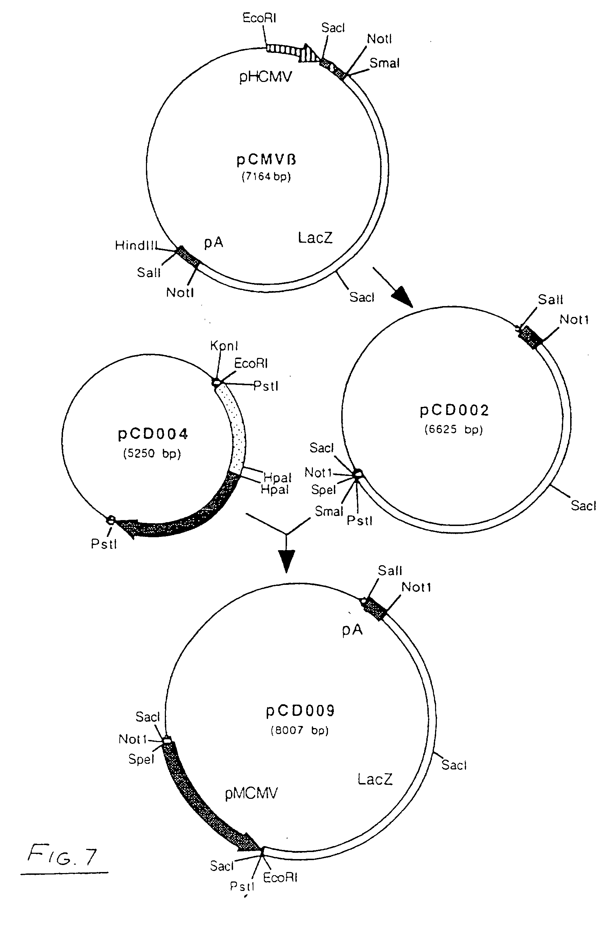

Le plasmide pCMVβ (Clontech Cat# 6177-1, figure 7) a été digéré par *Sal*I et *Sma*I pour isoler le fragment *Sal*I*-Sma*I de 3679 pb contenant le gène *lacZ* ainsi que le signal de poly-adénylation du gène tardif du virus SV40. Ce fragment a été inséré dans le vecteur pBS-SK+, préalablement digéré par *Sal*I et *EcoRV,* pour donner le plasmide pCD002 de 6625 pb (figure 7). Ce plasmide contient le gène reporter *lacZ* mais aucun promoteur n'est situé en amont de ce gène.

Le virus MCMV souche Smith a été obtenu de l'American Type Culture Collection, Rockville, Maryland, USA (ATCC N° VR-194). Ce virus a été cultivé sur cellules d'embryon de souris Balb/C et l'ADN viral de ce virus a été préparé comme décrit par Ebeling A. et al. (J. Viral. 1983. **47**. 421-433). Cet ADN génomique viral a été digéré par *Pst*I pour isoler le fragment *Pst*I*-Pst*I de 2285 pb. Ce fragment a été cloné dans le vecteur pBS-SK+, préalablement digéré par *Pst*I et traité avec la phosphatase alcaline. pour donner le plasmide pCD004 (figure 7). Le plasmide pCD004 a été digéré par *Hpa*I et *Pst*I pour isoler le fragment *Hpa*I*-Pst*I de 1389 pb qui contient la région promotrice/activatrice du gène Immediate-Early du cytomégalovirus murin (Murine CytoMegaloVirus = MCMV) (Dorsch-Häsler K. *et al.* Proc. Natl. Acad. Sci. 1985. **82**. 8325-8329, et demande de brevet WO-A-87/03905). Ce fragment a été cloné dans le plasmide pCD002, préalablement digéré par *Pst*I et *Sma*I, pour donner le plasmide pCD009 de 8007 pb (figure 7).

Un oligonucléotide double brin a été obtenu par hybridation des deux oligonucléotides suivants :

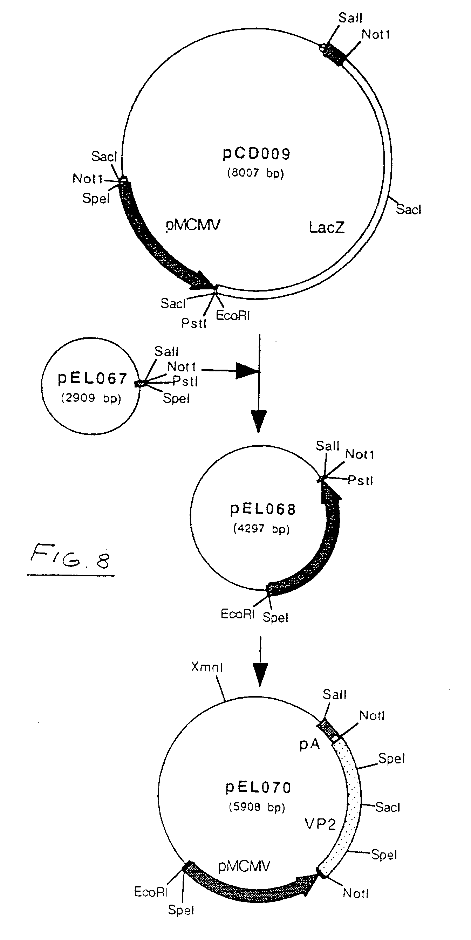

Cet oligonucléotide double brin a été ligaturé avec le vecteur pBS-SK+, préalablement digéré par KpnI et *Sac*I, pour donner le plasmide pEL067 (figure 8).

Le plasmide pCD009 a été digéré par *Pst*I et *Spe*I pour isoler le fragment *Pst*I*-Spe*I de 1396 pb. Ce fragment a été ligaturé avec le plasmide pEL067, préalablement digéré par *Pst*I et *Spe*I, pour donner le plasmide pEL068 de 4297 pb (figure 8). Le plasmide pEL024 (voir exemple 6, paragraphe 6.1 et figure 5) a été digéré par *Hind*III et *Not*I pour isoler le fragment *Hind*III*-Not*I de 1390 pb (fragment A). Le plasmide pEL027 (voir exemple 6, paragraphe 6.1 et figure 5) a été digéré par *Hind*III et *Sal*I pour isoler le fragment *Hind*III-*Sal*I de 235 pb (fragment B). Les fragments A et B ont été ligaturés ensemble avec le plasmide pEL068, préalablement digéré par *Not*I et *Sal*I, pour donner le plasmide pEL070 de 5908 pb (figure 8). Ce plasmide contient donc une cassette d'expression constituée du promoteur IE du MCMV, du gène VP2 et du signal polyA de SV40.

### 7.2 - Construction du plasmide donneur pEL170

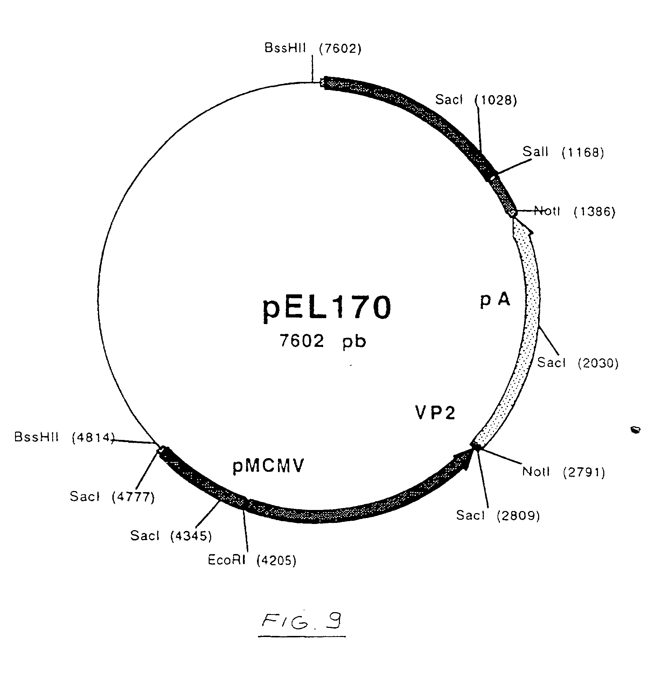

Le plasmide pEL070 a été digéré par *Eco*RI, *Sal*I et *Xmn*I pour isoler le fragment *Eco*RI*-Sal*I de 3035 pb. Ce fragment a été ligaturé dans le plasmide pEL168 (voir exemple 5 et figure 3), préalablement digéré par *Eco*RI et *Sal*I, pour donner le plasmide pEL170 de 7602 pb (figure 9). Ce plasmide permet l'insertion de la cassette d'expression MCMV-IE/IBDV-VP2 dans le site intergénique entre les COLs D et E du virus ILTV.

### 7.3 - Isolement et purification du virus recombinant vILTV14

Le virus vILTV14 a été isolé et purifié après cotransfection de l'ADN du plasmide pEL170 préalablement linéarisé par l'enzyme *Bss*HII et de l'ADN viral, comme décrit dans l'exemple 3. Ce recombinant contient une cassette MCMV-IE/IBDV VP2 dans le site intergénique entre les COLs D et E du virus ILTV (voir exemple 5).

### Exemple 8: Construction du plasmide donneur pEL171 pour l'insertion d'une cassette d'expression du gène HN du NDV dans le site intergénique entre les COLs D et E et isolement de vILTV15:

### 8.1 - Clonage du gène HN du virus de la maladie de Newcastle (NDV)

La constitution d'une banque d'ADN complémentaire du génome du virus de la maladie de Newcastle (NDV), souche Texas, a été réalisée comme décrit par Taylor J. *et al*. (J. Virol. 1990. **64.** 1441-1450). Un clone pBR322 contenant la fin du gène fusion (F), la totalité du gène hémagglutinine-neuraminidase (HN) et le début du gène de la polymérase a été identifié pHN01. La séquence du gène NDV HN contenue sur ce clone est présentée sur la figure 10 (SEQ ID NO:8). Le plasmide pHN01 a été digéré par *Sph*I et *Xba*I pour isoler le fragment *Sph*I*-Xba*I de 2520 pb. Ce fragment a été ligaturé avec le vecteur pUC19, préalablement digéré par *Sph*I et *Xba*I, pour donner le plasmide pHN02 de 5192 pb. Le plasmide pHN02 a été digéré par *Cla*I et *Pst*I pour isoler le fragment *Cla*I*-Pst*I de 700 pb (fragment A). Une PCR a été réalisée avec les oligonucléotides suivants: et la matrice pHN02 pour produire un fragment PCR de 270 pb. Ce fragment a été digéré par *Hind*III et *Pst*I pour isoler un fragment *Hind*III*-Pst*I de 220 pb (fragment B).

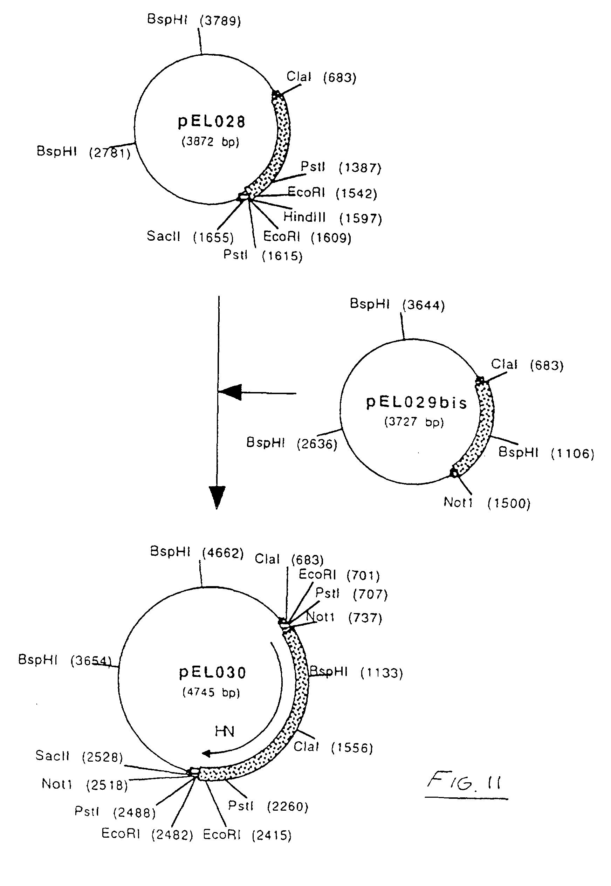

Les fragments A et B ont été ligaturés ensemble avec le vecteur pBS-SK+, préalablement digéré par *Cla*I et *Hind*III, pour donner le plasmide pEL028 de 3872 pb (figure 11). Le plasmide pHN02 a été digéré par *Bsph*I et *Cla*I pour isoler le fragment *Bsph*I*-Cla*I de 425 pb (fragment C). Une PCR a été réalisée avec les oligonucléotides suivants: et la matrice pHN02 pour produire un fragment PCR de 465 pb. Ce fragment a été digéré par *Bsph*I et *Not*I pour isoler le fragment *Bsph*I*-Not*I de 390 pb (fragment D). Les fragments C et D ont été ligaturés ensemble avec le vecteur pBS-SK+, préalablement digéré par *Cla*I et *Not*I, pour donner le plasmide pEL029bis de 3727 pb (figure 11). Le plasmide pEL028 a été digéré par *Cla*I et *Sac*II pour isoler le fragment *Cla*I-*Sac*II de 960 pb (fragment E). Le plasmide pEL029bis a été digéré par *Cla*I et *Not*I pour isoler le fragment *Cla*I*-Not*I de 820 pb (fragment F). Les fragments E et F ont été ligaturés ensemble avec le vecteur pBS-SK+, préalablement digéré par *Not*I et *Sac*II, pour donner le plasmide pEL030 de 4745 pb (figure 11).

### 8.2 - Construction du plasmide pEL171 contenant une cassette d'expression de HN du NDV dans le site intergénique entre les COLs D et E

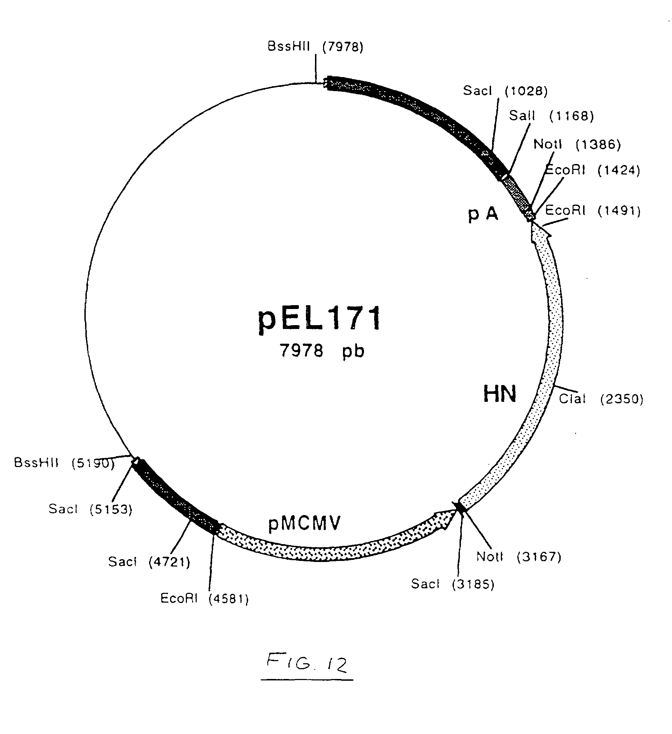

Le plasmide pEL030 a été digéré par *Not*I pour isoler le fragment *Not*I*-Not*I de 1780 pb (gène NDV HN entier). Ce fragment a été inséré dans les sites *Not*I du plasmide pEL170 (exemple 7, figure 9) à la place du fragment *Not*I*-Not*I de 1405 pb contenant le gène codant pour la protéine VP2 de l'IBDV; ce clonage a permis d'isoler le plasmide pEL171 de 7978 pb (figure 12). Ce plasmide permet l'insertion de la cassette d'expression MCMV-IE/NDV-HN dans le site intergénique entre les COLs D et E du virus ILTV.

### 8.3 - Isolement et purification du virus recombinant vILTV15

Le virus vILTV15 a été isolé et purifié après cotransfection de l'ADN du plasmide pEL171 préalablement linéarisé par l'enzyme *Bss*HII et de l'ADN viral, comme décrit dans l'exemple 3. Ce recombinant contient une cassette MCMV-IE/NDV HN dans le site intergénique entre les COLs D et E du virus ILTV (voir exemple 5).

### Exemple 9: Construction du plasmide donneur pEL172 pour l'insertion d'une cassette d'expression du gène F du NDV dans le site intergénique entre les COLs D et E et isolement de vILTV16:

### 9.1 - Clonage du gène F du virus de la maladie de Newcastle (NDV)

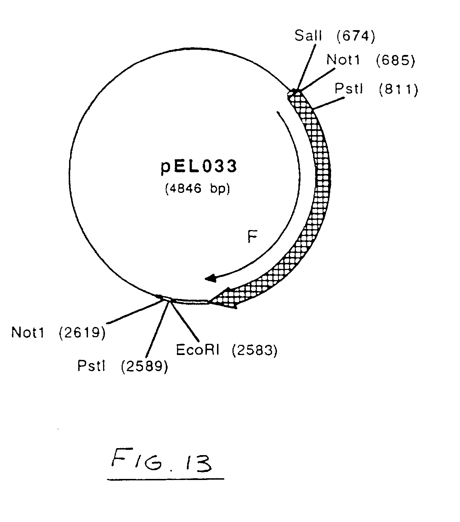

Un clone provenant de la banque d'ADN complémentaire du génome du virus de la maladie de Newcastle (voir exemple 8, paragraphe 8.1) et contenant le gène fusion (F) en entier a été appelé pNDV81. Ce plasmide a été décrit précédemment et la séquence du gène NDV F présent sur ce clone a été publiée (Taylor J. *et al.* J. Virol., 1990, **64,** 1441-1450). Le plasmide pNDV81 a été digéré par *Nar*I et *Pst*I pour isoler le fragment *Nar*I-*Pst*I de 1870 pb (fragment A). Une PCR a été réalisée avec les oligonucléotides suivants: et la matrice pNDV81 pour produire un fragment de 160 pb. Ce fragment a été digéré par *Pst*I et *Sal*I pour isoler le fragment *Pst*I*-Sal*I de 130 pb (fragment B). Les fragments A et B ont été ligaturés ensemble avec le vecteur pBS-SK+, préalablement digéré par *Cla*I et *Sal*I, pour donner le plasmide pEL033 de 4846 pb (figure 13).

### 9.2 - Construction du plasmide pEL172 contenant une cassette d'expression du gène F du NDV dans le site intergénique entre les COLs D et E

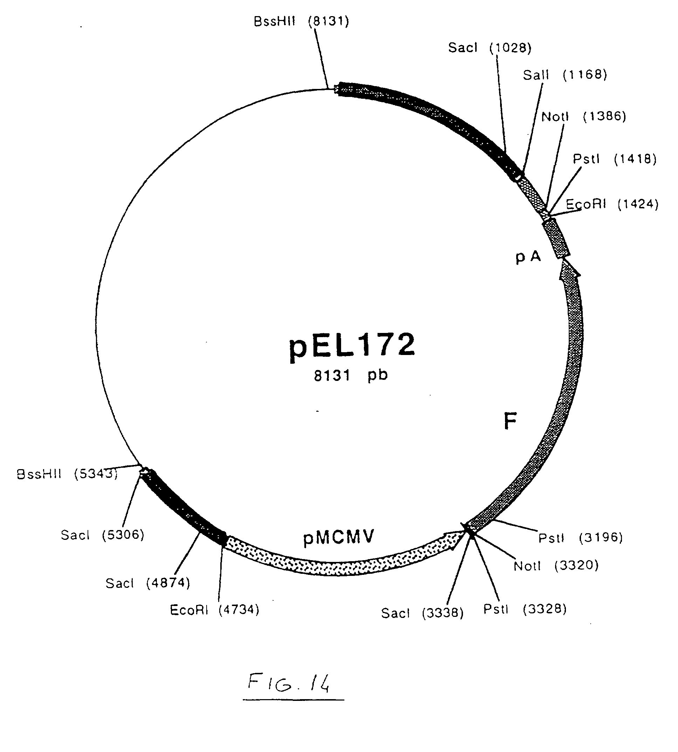

Le plasmide pEL033 a été digéré par *Not*I pour isoler le fragment *Not*I*-Not*I de 1935 pb (gène F entier). Ce fragment a été inséré dans les sites *Not*I du plasmide pEL170 (exemple 7, figure 9) à la place du fragment *Not*I*-Not*I de 1405 pb contenant le gène codant pour la protéine VP2 de l'IBDV; ce clonage a permis d'isoler le plasmide pEL172 de 8131 pb (figure 14). Ce plasmide permet l'insertion de la cassette d'expression MCMV-IE/NDV-F dans le site intergénique entre les COLs D et E du virus ILTV.

### 9.3 - Isolement et purification du virus recombinant vILTV16

Le virus vILTV16 a été isolé et purifié après cotransfection de l'ADN du plasmide pEL172 préalablement linéarisé par l'enzyme *Bss*HII et de l'ADN viral, comme décrit dans l'exemple 3. Ce recombinant contient une cassette MCMV-IE/NDV F dans le site intergénique entre les COLs D et E du virus ILTV (voir exemple 5).

### Exemple 10: Construction d'un plasmide donneur pour l'insertion d'une double cassette d'expression des gènes HN et F du NDV dans le site intergénique entre les COLs D et E et isolement d'un virus recombinant ILTV:

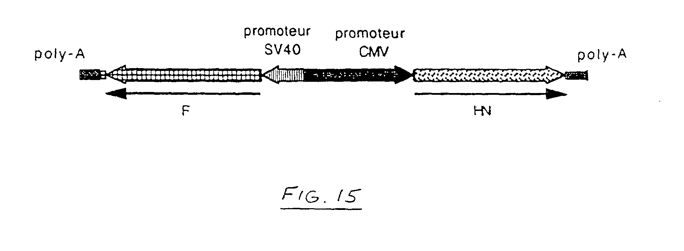

Une double cassette d'expression de deux gènes, par exemple les gènes HN et F du virus NDV, peut être construite. Une telle construction est schématisée à la figure 15. Dans cette construction, l'extrémité 5' des deux promoteurs sont adjacentes de manière que la transcription des deux gènes se fasse en sens opposés. Un des deux promoteurs est le promoteur MCMV IE et l'autre promoteur (appelé promoteur associé) est le promoteur SV40 (présent dans le plasmide pSVbeta, Clontech Laboratories, Palo Alto, California 94303-4607, USA). Dans cette configuration, le promoteur associé est activé par la région activatrice du promoteur CMV IE.

Cette double cassette d'expression peut ensuite être insérée dans le plasmide donneur décrit ci-dessus (pEL168 décrit dans l'exemple 5 et représenté dans la figure 3).

L'isolement des virus recombinants se fait de la même manière que ci-dessus (voir exemple 3).

### Exemple 11: Construction du plasmide donneur pEL181 pour l'insertion d'une cassette d'expression du gène gB du MDV dans le site intergénique entre les COLs D et E et-isolement de vILTV17:

### 11.1 - Clonage du gène gB du virus de la maladie de Marek

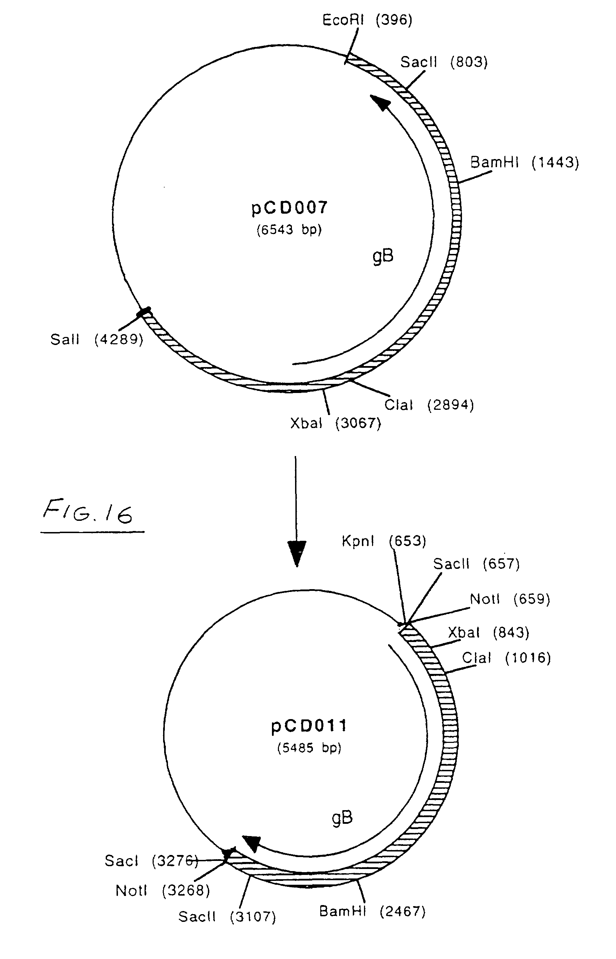

Le fragment *Eco*RI*-Sal*I de 3,9 kpb de l'ADN génomique du virus MDV souche RB1B contenant le gène MDV gB (séquence publiée par Ross N. *et al*. J. Gen. Virol. 1989. **70,** 1789-1804) a été ligaturé avec le vecteur pUC13, préalablement digéré par *Eco*RI et *Sal*I, pour donner le plasmide pCD007 de 6543 pb (figure 16). Ce plasmide a été digéré par *Sac*I et *Xba*I pour isoler le fragment *Sac*I*-Xba*I de 2260 pb (partie centrale du gène gB = fragment A). Une PCR a été réalisée avec les oligonucléotides suivants: et la matrice pCD007 pour produire un fragment PCR de 222 pb. Ce fragment a été digéré par *Kpn*I et *Xba*I pour isoler un fragment *Kpn*I*-Xba*I de 190 pb (extrémité 5' du gène gB = fragment B). Une autre PCR a été réalisée avec les oligonucléotides suivants: et la matrice pCD007 pour produire un fragment PCR de 195 pb. Ce fragment a été digéré par *Sac*I et *Sac*II pour isoler le fragment *Sac*I*-Sac*II de 162 pb (extrémité 3' du gène gB = fragment C). Les fragments A, B et C ont été ligaturés ensemble avec le vecteur pBS-SK+, préalablement digéré par *Kpn*I et *Sac*I, pour donner le plasmide pCD011 de 5485 pb (figure 16).

### 11. 2 - Construction du plasmide pEL181 contenant une cassette d'expression du gène gB du MDV dans le site intergénique entre les COLs D et E du virus ILTV

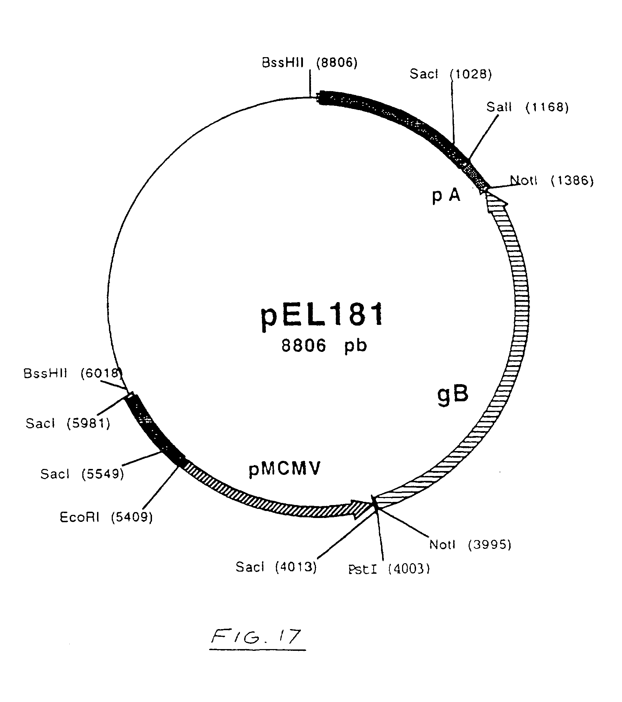

Le plasmide pCD01 a été digéré par *Not*I pour isoler le fragment *Not*I*-Not*I de 2608 pb (gène gB MDV entier). Ce fragment a été inséré dans les sites *Not*I du plasmide pEL170 exemple 7, figure 9) à la place du fragment *Not*I*-Not*I de 1405 pb contenant le gène codant pour la protéine VP2 de l'IBDV; ce clonage a permis d'isoler le plasmide pEL181 de 8806 pb (figure 17). Ce plasmide permet l'insertion de la cassette d'expression MCMV-IE/MDV-gB dans le site intergénique entre les COLs D et E du virus ILTV.

### 11.3 - Isolement et purification du virus recombinant vILTV17

Le virus vILTV17 a été isolé et purifié après cotransfection de l'ADN du plasmide pEL181 préalablement linéarisé par l'enzyme *Bss*HII et de l'ADN viral, comme décrit dans l'exemple 3. Ce recombinant contient une cassette MCMV-IE/MDV gB dans le site intergénique entre les COLs D et E du virus ILTV (voir exemple 5).

### Exemple 12: Construction d'un plasmide donneur pour l'insertion d'une cassette d'expression de gène(s) de l'IBV dans le site intergénique entre les COLs D et E et isolement de virus recombinant ILTV:

Selon la même stratégie que celle décrite plus haut pour l'insertion de simples cassettes (exemples 6, 7, 8, 9 et 11) ou pour l'insertion de doubles cassettes (exemple 10), dans le site décrit ci-dessus (exemple 5), il est possible de réaliser des virus ILTV recombinants exprimant à un niveau élevé les protéines Membrane (M) ou Spike (S), ou partie de Spike (S1 ou S2), ou Nucléocapside (N) du virus de la bronchite infectieuse aviaire (IBV). On réalise notamment une double cassette d'expression avec le gène S sous controle du promoteur CMV lE et le gène M sous contrôle du promoteur associé.

### Exemple 13: Construction de plasmides donneurs pour l'insertion de cassettes d'expression de gène(s) d'autres agents pathogènes aviaires ou de peptide immunomodulateur dans le site décrit et isolement de virus recombinants ILTV:

Selon la même stratégie que celle décrite plus haut pour l'insertion de simples cassettes (exemples 6, 7, 8, 9 et 11), ou pour l'insertion de doubles cassettes (exemple 10), dans le site décrit ci-dessus (exemple 5), il est possible de réaliser des virus ILTV recombinants exprimant à un niveau élevé des immunogènes du CAV (et notamment une double cassette d'expression des gènes codant pour VP1 et pour VP2), du virus de la pneumonivirose du poulet, ou d'autres agents pathogènes aviaires, ou encore des peptides immunomodulateurs et notamment des cytokines.

### Exemple 14: Production de vaccins:

Les virus recombinants obtenus selon l'invention sont produits sur oeufs embryonnés. La solution virale récoltée est ensuite diluée dans une solution stabilisatrice pour la lyophilisation, répartie à raison de 1000 doses vaccinales par flacon, et enfin lyophilisée

## Revendications

1. Un virus ILTV comprenant et exprimant au moins une séquence nucléotidique hétérologue, cette séquence nucléotidique étant insérée dans le locus d'insertion formé par l'intergène situé entre les codons stop des COL D et COL E d'ILTV et qui, dans la souche d'ILTV T-20 12-8-66 est défini entre les nucléotides 3873 et 4260 à la SEQ ID NO:1.

2. Le virus selon la revendication 1, dans lequel la ou les séquences nucléotidiques sont insérées par insertion simple, ou après délétion totale ou partielle du locus d'insertion.

3. Le virus selon l'une quelconque des revendications 1 à 2, qui comprend un promoteur d'origine virale ou cellulaire pour exprimer la séquence nucléotidique insérée.

4. Le virus selon la revendication 3, dans lequel le promoteur est choisi parmi le groupe consistant en: promoteur CMV immediate-early, promoteur LTR du virus du Sarcome de Rous (RSV), promoteur précoce du virus SV40.

5. Le virus selon l'une quelconque des revendications 1 à 4, qui comprend au moins deux séquences nucléotidiques insérées dans le locus d'insertion sous le contrôle de promoteurs différents.

6. Le virus selon la revendication 5, dans lequel les promoteurs sont des promoteurs immediate-early de virus CMV d'origines animales différentes.

7. Le virus selon la revendication 5, qui comprend une première séquence nucléotidique associée au promoteur CMV immediate early et un autre promoteur sous la dépendance duquel se trouve une autre séquence nucléotidique, ces deux promoteurs étant disposés de manière que leurs extrémités 5' soient adjacentes.

8. Le virus selon l'une quelconque des revendications 1 à 7, qui comprend, insérée dans le locus d'insertion, une cassette d'expression comprenant successivement un promoteur, deux ou plusieurs gènes séparés deux à deux par un IRES, et un signal de polyadénylation.

9. Le virus selon l'une quelconque des revendications 1 à 8, qui comprend une séquence nucléotidique codant pour un polypeptide antigénique d'un agent pathogène aviaire, cette séquence étant insérée dans le locus d'insertion.

10. Le virus selon la revendication 9, qui comprend une séquence codant pour un antigène d'un agent pathogène aviaire choisi parmi le groupe consistant en le virus de la maladie de Newcastle (NDV), le virus de la maladie de Gumboro (IBDV), le virus de la maladie de Marek (MDV), le virus de la bronchite infectieuse (IBV), le virus de l'anémie du poulet (CAV), le virus de la pneumovirose du poulet.

11. Le virus selon la revendication 10, qui comprend, une séquence nucléotidique, choisie parmi les séquences nucléotidiques codant pour les polypeptides F et/ou HN du virus NDV.

12. Le virus selon la revendication 10, qui comprend une séquence nucléotidique, choisie parmi les séquences nucléotidiques codant pour les polypeptides gB, gC, gD, gH+gL du virus MDV.

13. Le virus selon la revendication 10, qui comprend au moins une séquence nucléotidique choisie parmi le groupe des séquences correspondant aux antigènes VP2 de l'IBDV, aux antigènes S, ou partie de S, M et N du virus IBV, aux antigènes VP1 et VP2 du CAV, aux antigènes G et F du virus de la pneumovirose du poulet.

14. Le virus selon l'une quelconque des revendications 1 à 13, qui comprend une séquence nucléotidique codant pour un polypeptide immunomodulateur, cette séquence étant insérée dans le locus d'insertion.

15. Le virus selon la revendication 14, dans lequel cette séquence nucléotidique code pour une cytokine.

16. Vaccin comprenant un virus ILTV selon l'une quelconque des revendications 1 à 15.

17. Formule de vaccin multivalent comprenant, en mélange ou à mélanger, au moins deux virus tels que définis dans l'une quelconque des revendications 1 à 15, ces virus comprenant des séquences insérées différentes.

## Patentansprüche

1. ILTV-Virus, der mindestens eine heterologe Nucleotidsequenz umfasst und exprimiert, wobei die Nucleotidsequenz in die Insertionsstelle eingefügt ist, die durch das Zwischengen gebildet wird, das sich zwischen den COL D- und COL E-Stopcodons von ILTV befindet und das im ITLV-Stamm T-20 12-8-66 zwischen den Nucleotiden 3873 und 4260 von SEQ ID NO.: 1 bestimmt ist.

2. Virus nach Anspruch 1, wobei die Nucleotidsequenz(en) durch eine einfache Insertion oder nach einer kompletten oder teilweisen Deletion der Insertionsstelle eingefügt sind.

3. Virus nach Anspruch 1 oder 2, das einen Promotor viralen oder zellulären Ursprungs umfasst, zur Expression der eingefügten Nucleotidsequenz.

4. Virus nach Anspruch 3, wobei der Promotor ausgewählt ist aus der Gruppe bestehend aus dem unmittelbar frühen CMV-Promotor, dem LTR-Promotor des Rous-Sarkom-Virus (RSV), dem frühen Promotor des SV40-Virus.

5. Virus nach einem der Ansprüche 1 bis 4, das mindestens zwei Nucleotidsequenzen umfasst, die in die Insertionsstelle unter der Kontrolle von verschiedenen Promotoren eingefügt sind.

6. Virus nach Anspruch 5, wobei die Promotoren Promotoren des unmittelbar frühen CMV-Virus aus verschiedenen tierischen Quellen sind.

7. Virus nach Anspruch 5, das eine erste Nucleotidsequenz, die mit dem unmittelbar frühen CMV-Promotor verknüpft ist, und das einen anderen Promotor umfasst, von dem eine andere Nucleotidsequenz abhängt, wobei die zwei Promotoren so angeordnet sind, dass ihre 5'-Enden benachbart sind.

8. Virus nach einem der Ansprüche 1 bis 7, das eine Expressionskassette eingefügt in die Insertionsstelle umfasst, die hintereinander einen Promotor, zwei oder mehr Gene, die jeweils durch einen IRES getrennt sind, und ein Polyadenylierungssignal umfasst.

9. Virus nach einem der Ansprüche 1 bis 8, das eine Nucleotidsequenz umfasst, die ein antigenes Polypeptid eines Vogelpathogens codiert, wobei die Sequenz in die Insertionsstelle eingefügt ist.

10. Virus nach Anspruch 9, das eine Sequenz umfasst, die ein Antigen eines Vogelpathogens codiert, das ausgewählt ist aus der Gruppe bestehend aus dem Virus der Newcastle-Krankheit (NDV), dem Virus der infektiösen Bursitis (IBDV), dem Virus der Marek-Krankheit (MDV), dem Virus der infektiösen Bronchitis (IBV), dem Virus der Geflügelanämie (CAV), dem Virus der Geflügel-Pneumoviruserkrankung.

11. Virus nach Anspruch 10, das eine Nucleotidsequenz umfasst, die ausgewählt ist aus den Nucleotidsequenzen, die die Polypeptide F und/oder HN des NDV-Virus codieren.

12. Virus nach Anspruch 10, das eine Nucleotidsequenz umfasst, die ausgewählt ist aus den Nucleotidsequenzen, die die Polypeptide gB, gC, gD, gH + gL des MDV-Virus codieren.

13. Virus nach Anspruch 10, das mindestens eine Nucleotidsequenz umfasst, die ausgewählt ist aus der Gruppe von Sequenzen, die den VP2-Antigenen von IBDV, den S-Antigenen oder einem Teil der S-, M- und N-Antigene des IBV-Virus, den VP1- und VP2-Antigenen von CAV, den G- und F-Antigenen des Virus der Geflügel-Pneumoviruserkrankung entsprechen.

14. Virus nach einem der Ansprüche 1 bis 13, das eine Nucleotidsequenz umfasst, die ein immunmodulatorisches Polypeptid codiert, wobei die Sequenz in die Insertionsstelle eingefügt ist.

15. Virus nach Anspruch 14, wobei die Nucleotidsequenz ein Cytokin codiert.

16. Impfstoff, umfassend ein ILTV-Virus nach einem der Ansprüche 1 bis 15.

17. Multivalente Impfstoffformulierung, umfassend mindestens zwei Viren wie in einem der Ansprüche 1 bis 15 definiert als Gemisch oder zum Mischen, wobei die Viren verschiedene eingefügte Sequenzen umfassen.

## Claims

1. An ILTV virus comprising and expressing at least one heterologous nucleotide sequence, this nucleotide sequence being inserted into the insertion locus formed by the intergene situated between the ILTV stop codons COL D and COL E and which, in the ILTV strain T-20 12-8-66, is defined between the nucleotides 3873 and 4260 in SEQ ID NO: 1.

2. The virus according to claim 1, in which the nucleotide sequence or sequences are inserted by simple insertion, or after total or partial deletion of the insertion locus.

3. The virus according to any one of claims 1 to 2, which comprises a promoter of viral or cellular origin in order to express the nucleotide sequence inserted.

4. The virus according to claim 3, in which the promoter is chosen from the group consisting of: CMV immediate-early promoter, LTR promoter of the Rous Sarcoma Virus (RSV), early promoter of the SV40 virus.

5. The virus according to any one of claims I to 4, which comprises at least two nucleotide sequences inserted into the insertion locus under the control of different promoters.

6. The virus according to claim 5, in which the promoters are CMV immediate-early promoters of the CMV virus of different animal origins.

7. The virus according to claim 5, which comprises a first nucleotide sequence associated with the CMV immediate-early promoter and another promoter on which another nucleotide sequence depends, these two promoters being arranged in such a way that their 5' ends are adjacent.

8. The virus according to anyone of claims 1 to 7, which comprises, inserted into the insertion locus, an expression cassette comprising successively a promoter, two or more genes separated two by two by an IRES, and a polyadenylation signal.

9. The virus according to any one of claims 1 to 8, which comprises a nucleotide sequence coding for an antigenic polypeptide of a pathogenic avian agent, this sequence being inserted into the insertion locus.

10. The virus according to claim 9, which comprises a sequence coding for an antigen of a pathogenic avian agent chosen from the group consisting of Newcastle Disease Virus (NDV). Gumboro Disease Virus (IBDV), Marek's Disease Virus (MDV), Infectious Bronchitis Virus (IBV), Chicken Anaemia Virus (CAV), chicken pneumovirus.

11. The virus according to claim 10, which comprises a nucleotide sequence, chosen from the nucleotide sequences coding for the F and/or HN polypeptides of the NDV virus.

12. The virus according to claim 10, which comprises a nucleotide sequence, chosen from the nucleotide sequences coding for the gB, gC, gD, gH + gL polypeptides of the MDV virus.

13. The virus according to claim 10, which comprises at least one nucleotide sequence, chosen from the group of sequences corresponding to the VP2 antigens of IBDV, to the S antigens, or S, M and N part of the IBV virus, to the VP1 and VP2 antigens, to the G and F antigens of the chicken pneumovirus.

14. The virus according to any one of claims 1 to 13, which comprises a nucleotide sequence coding for an immunomodulating polypeptide, this sequence being inserted into the insertion locus.

15. The virus according to claim 14, in which this nucleotide sequence codes for a cytokine.

16. Vaccine comprising an ILTV virus according to any one of claims 1 to 15.

17. Multivalent vaccine formula comprising, mixed or to be mixed, at least two viruses as defined in any one of claims 1 to 15, these viruses comprising different inserted sequences.


### LISTE DE SEQUENCES

(1) INFORMATIONS GENERALES:
   (i) DEPOSANT:
      (A) NOM: MERIAL
      (B) RUE: 17 rue Bourgelat
      (C) VILLE: LYON
      (E) PAYS: FRANCE
      (F) CODE POSTAL: 69002
   (ii) TITRE DE L' INVENTION: Vaccin vivant recombinant aviaire utilisant comme vecteur le virus de la laryngotracheite infectieuse aviaire
   (iii) NOMBRE DE SEQUENCES: 19
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (v) DONNEES DE LA DEMANDE ACTUELLE: NUMERO DE LA DEMANDE: FR 98/00122
   (vi) DONNEES,DE LA DEMANDE ANTERIEURE:
      (A) NUMERO DE LA DEMANDE: FR 97/01317
      (B) DATE DE DEPOT: 31-JAN-1997
(2) INFORMATIONS POUR LA SEQ ID NO: 1:
   (i) CARACTERISTIQUES DE LA SEQUENCE:
      (A) LONGUEUR: 7082 paires de bases
      (B) TYPE: nucléotide
      (C) NOMBRE DE BRINS: double
      (D) CONFIGURATION: linéaire
   (ii) TYPE DE MOLECULE: ADN (génomique)
   (iii) HYPOTHETIQUE: NON
   (iv) ANTI-SENS: NON
   (vi) ORIGINE:
      (A) ORGANISME: Infectious Laryngotracheitis Virus
      (B) SOUCHE: T20
   (xi) DESCRIPTION DE LA SEQUENCE: SEQ ID NO: 1:
   GAATTCTAAG CTTGCTTTGT CGAACCAGTT TTGTTTTCTT TTTGGGGGGA GGGTAGCACA    60
   CTCTGCCCGA GTCTCGGCAT TGACTTAACA GTGATGTGAA ACCCGGAAGA TCGAGCATGA   120
   ACTAATAGCA TTAAAGAATT GTTATCCGAG GAATAATCGT GGACGCGAAT TTACTCGACC   180
   GCTAAAATCT TTCTTCTACT GAGCTGGATA CGTGAAATTT GGTGAGTATA ACCTCTCGGG   240
   ATACATAGCT TTTAAATACG GGGCGTGCAA TATTAAATTC TGCACTCGGG GCTGCAATGG   300
   AGCGCGGAGC TTTATTTGAC AAGACCGCCA ATTGCAAGGA CTGGGTCTCG ATCGGGACTA   360
   CTGTGTGGGG CGCGATCGAT GCAGATGACG GGGACGACTT AGTCTGGGAT TATGAAAATA   420
   GCCCATATCC AAGCATAGTT TCCTCACTAT TCCCGGGGGA AGAAACGGAC TGGGCAATTT 480
   GTAACTCTGT TGTTGCCGCA AACCCCTGTA GCATACCTCC TGGGCGGCAG CGTTTGGCAT 540
   GGCCATGCTG CTGTTTTCGT CGGCCAGACA GCTTCTCCGT CCCGCGCGTG GAAGTTAATG 600
   CTCGCCTTGT TGCCGCGGTT GCACTGATAA TTTTCTCATT GCTTGTAGTG ATCTGTGTTG 660
   CGTCATATTG GGGGTAACAT GTCTTCAGAG GACACATCGG GATTCCTAAC GCCCCCCGCA 720
   AGTGATGACG ACACTGACCC TTCCGAGCCA CCACCAAATT TATGGGATCC TCACCAGGAC 780
   GATTTTCCGA GGGACGCTGA TTCCCCAAAC CCACTTTTCT ACCCCTGGGA TGACTCTGTG 840
   AATAATACTG GGGATACGGG CAGTAACGAA GATGACTATG TAGATATGGG AGGGGTAGGT 900
   GGATCCGAAG ACTATGAAGA CCTCGGTACG GGCGGGGACT CTGACTATGA CAATGTATCT 960
   ACAGCGACCG GCGGGACGTG GTTTCCTTCC CTTACTTCTT GGTCATCAGA GGACCACGGC 1020
   CCAACTTCTC CGGAAAACCC TATGCAACAA CTTCAAGTAA CAATTCAGCA GGATTCAGAT 1080
   CCACAGCAGG AACCCGATCC CCAGCAAGTT CCCGGTCTCC AGCAGGAACC TGACCCCCAG 1140
   CAAGATCCAC GAGAGCCTCG TGATCCTCCT CCCTATAGTC CGCCCCCAGA GGACCCTTTT 1200
   GGGCTCTCGC CATTTACTAG TGGGATGGGC GGGTTTGGGC CACCGTGGCG TGGCCCCAGC 1260
   CACCCTCGTA TGATGAGGCA ATGGGGGATG GACCTTTTAC TACGACTGGG GGTCGGCGAC 1320
   CTTGCTCACG CAGGCGCGGT CGTCGCCGGT CTCGAGGTCG ATCTCGGCGA CGCAATTGGT 1380
   GTGCAGCAAA GTTGTGCTCA GAGGCATGTT TGTGGCATCT CTTTTGGGTT GGAGTGGCCT 1440
   TAATGTGTTG GTGGCTCTTG TATCTTATTT TGCGCATTGT CTGGGGACAG ACTCCGGGAT 1500
   AAGGAAGGTT GTATCCGCAT CCAGTACTCC TCAATAAAAG CGTGGTGGTG CTACACGATG 1560
   TCTGTTAATT TTACAACTCC ATTTTACAGG TGATCTAGAG AGACGCTGAG TGGCACTTGT 1620
   CCCGACGGGA CCATGCAGTC GAACAGCAGC GATGAGGCCC AGTGTGATGA TGTCGAGGAG 1680
   GGATGGTCGT CCATAGCTCC AGGTGATGCA CTGGATACAG ATTTCATTCC AGGGCCTTGT 1740
   GCCACGTCCA TACATGGTAT ATCCAAGGCA GTTTATTTTT TTCTGTGTGG AGTTAATCTG 1800
   GAGGAATGTA GTACACTCCC ACAGCATGTC CAATCTCACC CATATGGACA TCCTGAGCTG 1860
   AAATCAGGCA AATGGTACAA GAGGTTTTGC TCCGGGCTAG GCGAAATTGG AGATACAAGC 1920
   CAGTGTCAGC TGACACGACT ATGCTGCACT TCCGGAATGC CGGCACAGAT TTTTGGGCCT 1980
   TCGAGATTCA GGTCTCTGCA ACAGAAGCCA ACCCATATGC GGGCCCAAGA TTTGCTCACT 2040
   AGGCCTTGCC ATATACTAGA GTTCGATGTT GGCGCTGACC TAATCAATCT TTTCTTGTAT 2100
   ATGGAACCAT GTTCAGGGAA TCGATATTGC GTACATTTAG GATACCATAA AACTAATGCC 2160
   ATGCGTGTTT TGAGCGGTGG TGGGATTCTA TGGGGCAGAC TTCCGTGGAA GGACAACACC 2220
   GAGGAGCACG GGTACTCGTT GCCTATGCGA GTATTTGGGA TCAAATTGCC CCATAAAGTT 2280
   TATGTGGCAT GTCGCTGCCC TGCAACTCGG ACGGAACTAT TATTTGGTGA GGGGGGGGTA 2340
   GGATTCAACG CGGAAAACTT TAAACAGTGC GGACGGTTGA AAAAAGAGTG TGAATGTCTG 2400
   CAGAAGGCTT GTTTTACTGC ACAAACGGTG TTAGGTGCGG CATGTAAGTT TACTGTATAC 2460
   TCGAGCAAGG GACGAGGTCA AGAAATTCTG CTATATCAGG ACCCATGAAT GCTACAACGG 2520
   TAATGCCTGT AGTACTGGGT ATGTTAAGTA GAGAACCCCA CAGGTGTGCA GGTACACTCA 2580
   TACTGTCCAG GTCCTCTGGA AATTGCCGTG GATTTCATGA GACCCAACAC GATATTCCCA 2640
   CTAACCCGGG TCTGTATCCT CTGTGTAATC ATGAGCACCC TTACTATGTG ACAGTTACAG 2700
   ATGTATGCGG CAACTGTTGT TCATGGCTTG AGCGGGTTTT TGGGAGAGTA GCTGCCCCTG 2760
   CTGGTCTAAG CTCCGTATCT GTATCCATTA AAGGCTCCAC CCACAGCGGG ACTGACGTGA 2820
   CAGAAGAACG TGAAGAGGAC TCAGGGACAC AGCAAACCTC CCACGACAAA TTGCCGGAGC 2880
   GCAACCGCAT GGGAGATCAA AATTCGAATT TGCGGGGAAG AGATCAATAT TGGCCGCCTG 2940
   CCCCACACCG TAGTCATTGT CACTCGGATT TTATATTCGA TGAACCTGAG CCAGAAAGTG 3000
   GGGAAGACGT GCATAACATG CATCCTCCAC GAGGTGCAGA TGAGCAAACA GCCGCTTCTG 3060
   TGTCAGCGCT AATGCAAAGT CTAGCACAAG CATTGGTGAG TGCACAAGCT ATTAGCAGCA 3120
   TGGTCTCTGG CTCTGCTTCC TCAGTGGGCG TAGAAGTAGA CTGTGGGTAC AGTCAGACTC 3180
   ATATTACAGA GGGGCCGGGG AGGGAACAAT TCGGTAGAGT CCCAGAAAGA GGGCCAGAGT 3240
   ATCCTCAAGA TTACTGTGAT ATATATGGTC CTGTAAGTAA TGGGCCTGCT GGATACAGAG 3300
   CAGGACCACC AGATGCTCCT AGTATACAAG ATAGGACCTT CCCATGCGGC AGAAGATGCG 3360
   ACGAAGCATG GCTTGCCTTA GAAGTAGGGA ATATGCCTTG GATTTCTTCT GGTTCACATA 3420
   GTCCACCTTC TCAGTATCAT AACCCTTATG GTTCACATAG TCCACCTTCC CAGTCTCATA 3480
   ACCCTTATGG TACATATAGT CCGCCTTCTC AGTCTCATAA CCCTTATGGC TCATATAGTC 3540
   CGCCTTCCCA GTATCATAAC CCTTGTGGTA CATATAGTCC GCCTTCTCAG TCTCGTAAGC 3600
   ATGACTATTC ACCTCCATAT CCGATACTCA AACCAAAGCC TCGATTACCC CCAGGCTTTG 3660
   AAAATACTGC TGGGATGTGG CCTCGATGTC CCCCTGGGTT TGAGGGGCGT CCATACAAAT 3720
   CTGGGGGCAT GGGTAACTTT CCTGGAAGTG CATGGACGGT AATAGATAGG GGGTCTAACC 3780
   AATGGCCAGC AGACGTGCGG GGGCCATTCT CAGATCAACG ATGGGCCCCC ACAGAGCATG 3840
   AAACGCGACG TTTTTGCGGG TATTACAGCT GAGCTCTCAT CATACCCATA ACTCCACTCA 3900
   TAACCCAAGG CCCATAAATC CATAACTCAT AACATAAATT CATACTTTCC GGTCGTCCAG 3960
   GGCACCACGT CATCAACAAG GATTGCAGAT AAATAAAAAT GCTCCACGTT GTCGGTGTCC 4020
   GTTGTATTGT ATTCTTTATT ATACCTCCGT AATTTTCGAG AGTCGGGGAA CATTCTAAAA 4080
   ATTTTAACCG TGCAATACTA CAGTGTATTT ACAAGGCCGG ATTGCAACAG TGAACTCATT 4140
   ACATCATTGA GCTCGCGGCG CCATCTGCTG ACCAGTCCAC AGAGATGGCA ATCTTCAGAA 4200
   ACGTAGGATG GCACCAATTC CAATACAATA CCGCCATCTG TCGATAGGTG TATAGAACTG 4260
   TCAAAACAAG TCGCAAGAGA AAAATTTCCC TACTGTATAC TGGCGGCTTA GCAGCTGCGC 4320
   ACAAACCACT CTGCATTCCT CTTTGCGGCA CACATTTGCG TGCTGCGCCA GAACGAGTGG 4380
   GATTTTTTTA GAACAGGTCC CAGGATAGTA CATGTCCCAC AATGTTCTCG CGGGGTCTAT 4440
   TGCTTTATGA TTCATGACCA TGGCCTCTGG TCGCGGATAC ACAATTCTTG AGAACCGGTC 4500
   GAAGAAGGTC AGTAATAAAG TTAAAGGACA TTTTGCGCTA CTCAGCGATA GCTCCTGAGA 4560
   TCTAGTGGTA TCTCTTAGTT GACTGCCAAT GCTAGAGAGA TAACACGGCA GGATTGGCCC 4620
   CAGATGCATG GCTAGAGATT GACATGCGCA GTAGATGTTA GAGAAGATAG GATCGTGGGG 4680
   GTAAATCCTT TCATCTTCGA ACTGATGCCA AAGCATCCAT ACAAGTGTCT CATCGCATGC 4740
   AAAAAGTAGC TCTTCAAATG AGCAGTTCGC CAAATATACA GCTCGTGAAA TTTTTGCCAA 4800
   CCTGGCTATA TCCGGACGCG ATGTCCAGCG GCCTTTCAGT GAAGCTGCGC GCCCACAAAA 4860
   CTGCTTCCAC GAAGTGAATG CAGCATCTGC TGCAAGGTCA GATGATCCCG AAGACAAAAA 4920
   TGCTGGAAAG CAGATTCCTC TATCACGATC GATATCATCA CAATCATCAT CATCCACTGC 4980
   CCGGTTTACC ATGTCTAAAA GACATTTCTG ATTTTCTAAT CTTAACTCTT CAGTAATGCA 5040
   CTTTCCGAGA CCGCCAAATG CAGTTGCGGC CTTTTCAAAA TATTGGGCCG GTGTTACGTT 5100
   TCGCAACTCC TTCGTTTCGG TCCGTGATGA CGTTGGGCAT CGGACAAAGT CTCTCCAAAT 5160
   CGGTCTTCGA AGTTCATCCC GATTTCTTTC CCAAGACCTG CGCGAATGCT TCAACGAAAC 5220
   AGTAAAGATA GGCGCCCTAT ATCGCTTTTC TGGTGTACCT GCACGGCGCC TGGGTCTAGG 5280
   GGGGATGCCT CTTCGGACTT GGATATGCGC ACGGCCTCTC ATAAACTTAT TTCGAGGGCT 5340
   ACTGACCCGC CTTATGGAAG ACCGTACGCT CATACCTGAC TGGTTCTCGT AACGGGAACG 5400
   AGGCTCGCCA TTCGAATAGC GACGGCGCCC CCTGTCACGT GAATCACGGA CTCGTTTGGA 5460
   GCTACCGCGC CCAGATGACC GGGATCTGGA GCTACTTTCT GTAGAATGAG ATCTGCGGCG 5520
   AATACAATGT GCTTCGTGGC GGGATTCTGA CCTCGAGCGT GAACGGCCAT CCAGGCGATC 5580
   TTTTGTCCTT TCCGTTGCTG ATCTCGCTTC GCTATGAGTA CTTGTATTGG AAGATGATCT 5640
   GGAACGTGTC TCACGCCTAT CTTTATTTAT TCCAGATTTC TCCGGTCTCC CCATCGCAGT 5700
   CAGTGGGTTG ATGTACCGCG TGCACGTCAA AAAAAATGAA ACCGCATACA CAACGGTTGA 5760
   GACTTCTACG GACTCAGAAC AGGTGTCGAG CTCGGAGCAG GTGCTGAGAG GTAAGCTGAC 5820
   AGTAATCTGG CACGCTGTTT GCGAGCTAAT CCACTTGGCT TTTGAATGGT CTGGGCCACT 5880
   CCCAGTATAC GTCATAACAC ATACACTGGA ACCCACAAAC TACAATTGCG GTCCAGTAGT 5940
   TGGTGCGAAA TATTCACGCA GACATAATAA TCTGCGAGAA CTTCTGCGGA TCCGACATGT 6000
   AACTTAATCC CGTAATGTAG TGCGGCATAC CGTCTAAACC GCAAACATCC GCTTAGTAGA 6060
   ACACGCCCTA AAATCACCCA CGAGTATACT TTGTACATTC TGACCGCCAG ATGTTACTCC 6120
   TTTCAAACAA TGATACTCAG CCGTTAGAAC TAGGGCTGTC TTCAAATGGA CCAAATTCAG 6180
   ACACAATACC GCACAACGTG TTTTAACATT TTATTGCCGT TCAAGGCCCG AACAATTTGT 6240
   TTTGTATCTT CTGTTCGTAT TTAAATGCAA TTATTACAAT GCTCGCAATC GCAGCCACGC 6300
   ACAATGCACG CAAGACTAAG CTCGAAGCAA TATTGGCAAG GCATGAAGTC AAGATACTGG 6360
   GAGGTTTCGA TGCGGAGACT TTAGTCTTTG CCGGAGTCGT GTAACCCATC AGTGATAAGA    6420
   GTTCTAGAAC CGCTGAGACA ATTACATATA CCCAAGCACT AATTTTTACG TGTTCATGTA    6480
   AAATATATGC ACACGGATCA TAGCAAATCC GCAATGTCAT GGTGGACATA ATAAGCGATG    6540
   CTAAATATAA ACCGATCTCG AGTGTTTTCA GTAGTAAGAC CCCTGGATTA CAGACGCAAA    6600
   ATCCTAGAGG GTCTGCTACG TAGTTCAAGC TTTTGCAATC TCGGAGATGT TCAAATTCCC    6660
   TCAAAAACCG TTGCATCTTT GTGTATGGCA CCCGTAATAT CAGCCTCGAA GTAAACGCTG    6720
   TCCAGTAGTT AAGATGATAT GCATCCATCG CAATGCGTTC TCGCGGCAGA CATAGCCCGA    6780
   ACATTCGCCT CTGGCGACGG GTCAGCAAGT AACATATGTA CAATACCGTC GAAATGAATG    6840
   CAACTCTGAT CTGGGAAAAC CATATGTAAA GCAGACAGTC TGCCACTTCC ATGACGAATA    6900
   CTGTTCTTTG GCCGGTGGGA TCAAGACGAG AAATGAGTAC AGTCTCATTT TCAAAATAAG    6960
   CTCCTTTAAC ACATCTCGTC TGGTGGTTCT CGTAGTTGTT TAGACGCTGT GTATATTTTG    7020
   TGAGGACATA AACTAGTGAA GAATTAAATG TAGCGGGGGG TTGGTGAACA GGATTGAATT    7080
   CC                                                                   7082
(2) INFORMATIONS POUR LA SEQ ID NO: 2:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (xi) DESCRIPTION DE LA SEQUENCE: SEQ ID NO: 2:
   TGCCGGAGCG CAACCGCATG G                                              21
(2) INFORMATIONS POUR LA SEQ ID NO: 3:
   (i) CARACTERISTIQUES DE LA SEQUENCE:
      (A) LONGUEUR: 59 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: OUI
   (xi) DESCRIPTION DE LA SEQUENCE: SEQ ID NO: 3:
   GACACCGAAT TCGTAAGCTT TCCCCGGGCA GTCGACAACG TGGAGCATTT TTATTTATC 59
(2) INFORMATIONS POUR LA SEQ ID NO: 4:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (xi) DESCRIPTION DE LA SEQUENCE: SEQ ID NO: 4:
   GTGTTATCTC TCTAGCATTG GC 22
(2) INFORMATIONS POUR LA SEQ ID NO: 5:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: OUI
   (xi) DESCRIPTION DE LA SEQUENCE: SEQ ID NO: 5:
   AGTTCTGAAT TCGTGTCCGT TGTATTGTAT TC 32
(2) INFORMATIONS POUR LA SEQ ID NO: 6:
   (i) CARACTERISTIQUES DE LA SEQUENCE:
      (A) LONGUEUR: 50 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: OUI
   (iv) ANTI-SENS: NON
   (xi) DESCRIPTION DE LA SEQUENCE: SEQ ID NO: 6:
   CGAATTCACT AGTGTGTGTC TGCAGGCGGC CGCGTGTGTG TCGACGGTAC    50
(2) INFORMATIONS POUR LA SEQ ID NO: 7 :
   (i) CARACTERISTIQUES DE LA SEQUENCE
      (A) LONGUEUR: 50 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: OUI
   (iv) ANTI-SENS: OUI
   (xi) DESCRIPTION DE LA SEQUENCE: SEQ ID NO: 7:
   CGTCGACACA CACGCGGCCG CCTGCAGACA CACACTAGTG AATTCGAGCT    50
(2) INFORMATIONS POUR LA SEQ ID NO: 8:
   (i) CARACTERISTIQUES DE LA SEQUENCE:
      (A) LONGUEUR: 2521 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (vi) ORIGINE:
      (A) ORGANISME: Newcastle disease virus
      (B) SOUCHE: Texas GB
   (xi) DESCRIPTION DE LA SEQUENCE: SEQ ID NO: 8:
   TGCTACCTGA TGTACAAGCA AAAGGCACAA CAAAAGACCT TGTTATGGCT TGGGAATAAT      60
   ACCCTTGATC AGATGAGAGC CACTACAAAA ATATGAATAC AAACGAGAGG CGGAGGTATC     120
   CCCAATAGCA ATTTGCGTGT AAATTCTGGC AACCTGTTAA TTAGAAGAAT TAAGAAAAAA     180
   CCACTGGATG TAAGTGACAA ACAAGCAATA CACGGGTAGA ACGGTCGGAG AAGCCACCCC     240
   TCAATCGGGA ATCAGGCCTC ACAACGTCCT TTCTACCGCA TCATCAATAG CAGACTTCGG     300
   TCATGGACCG TGCAGTTAGC AGAGTTGCGC TAGAGAATGA AGAAAGAGAA GCAAAGAATA     360
   CATGGCGCTT TGTATTCCGG ATTGCAATCT TACTTTTAAT AGTAACAACC TTAGCCATCT     420
   CTGCAACCGC CCTGGTATAT AGCATGGAGG CTAGCACGCC TGGCGACCTT GTTGGCATAC     480
   CGACTATGAT CTCTAAGGCA GAAGAAAAGA TTACATCTGC ACTCAGTTCT AATCAAGATG     540
   TAGTAGATAG GATATATAAG CAGGTGGCCC TTGAGTCTCC ATTGGCGTTG CTAAACACTG     600
   AATCTGTAAT TATGAATGCA ATAACGTCTC TCTCTTATCA AATCAATGGA GCTGCAAATA     660
   ATAGCGGGTG TGGGGCACCT GTTCATGACC CAGATTATAT CGGGGGGATA GGCAAAGAAC     720
   TTATTGTGGA TGACGCTAGT GATGTCACAT CATTCTATCC CTCTGCGTTC CAAGAACACC    780
   TGAACTTTAT CCCGGCACCT ACTACAGGAT CAGGTTGCAC TCGGATACCC TCATTCGACA    840
   TAAGCGCTAC CCACTACTGT TACACTCACA ATGTGATATT ATCTGGTTGC AGAGATCACT    900
   CACACTCATA TCAGTACTTA GCACTTGGCG TGCTTCGGAC ATCTGCAACA GGGAGGGTAT    960
   TCTTTTCTAC TCTGCGTTCC ATCAATTTGG ATGACAGCCA AAATCGGAAG TCTTGCAGTG   1020
   TGAGTGCAAC TCCCTTAGGT TGTGATATGC TGTGCTCTAA AATCACAGAG ACTGAGGAAG   1080
   AGGATTATAG TTCAATTACG CCTACATCGA TGGTGCACGG AAGGTTAGGG TTTGACGGTC   1140
   AATACCATGA GAAGGACTTA GACGTCATAA CTTTATTTAA GGATTGGGTG GCAAATTACC   1200
   CAGGAGTGGG GGGTGGGTCT TTTATTAACA ACCGCGTATG GTTCCCAGTC TACGGAGGGC   1260
   TAAAACCCAA TTCGCCTAGT GACACCGCAC AAGAAGGGAG ATATGTAATA TACAAGCGCT   1320
   ACAATGACAC ATGCCCAGAT GAACAAGATT ACCAGATTCG GATGGCTAAG TCTTCATATA   1380
   AGCCTGGGCG GTTTGGTGGA AAACGCGTAC AGCAGGCCAT CTTATCTATC AAGGTGTCAA   1440
   CATCTTTGGG CGAGGACCCG GTGCTGACTG TACCGCCTAA TACAATCACA CTCATGGGGG   1500
   CCGAAGGCAG AGTTCTCACA GTAGGGACAT CTCATTTCTT GTACCAGCGA GGGTCTTCAT   1560
   ACTTCTCTCC TGCTTTATTA TACCCTATGA CAGTCAACAA CAAAACGGCT ACTCTTCATA   1620
   GTCCTTACAC ATTCAATGCT TTCACTAGGC CAGGTAGTGT CCCTTGTCAG GCATCAGCAA   1680
   GATGCCCCAA CTCATGTGTC ACTGGAGTTT ATACTGATCC GTATCCCTTA GTCTTCCATA   1740
   GGAACCATAC CTTGCGGGGG GTATTCGGGA CAATGCTTGA TGATGAACAA GCAAGACTTA   1800
   ACCCTGTATC TGCAGTATTT GATAACATAT CCCGCAGTCG CATAACCCGG GTAAGTTCAA   1860
   GCCGTACTAA GGCAGCATAC ACGACATCGA CATGTTTTAA AGTTGTCAAG ACCAATAAAA   1920
   CATATTGCCT CAGCATTGCA GAAATATCCA ATACCCTCTT CGGGGAATTC AGGATCGTTC   1980
   CTTTACTAGT TGAGATTCTC AAGGATGATG GGATTTAAGA AGCTTGGTCT GGCCAGTTGA   2040
   GTCAACTGCG AGAGGGTCGG AAAGATGACA TTGTGTCACC TTTTTTTTGT AATGCCAAGG   2100
   ATCAAACTGG ATACCGGCGC GAGCCCGAAT CCTATGCTGC CAGTCAGCCA TAATCAGATA   2160
   GTACTAATAT GATTAGTCTT AATCTTGTCG ATAGTAACTT GGTTAAGAAA AAATATGAGT   2220
   GGTAGTGAGA TACACAGCTA AACAACTCAC GAGAGATAGC ACGGGTAGGA CATGGCGAGC   2280
   TCCGGTCCCG AAAGGGCAGA GCATCAGATT ATCCTACCAG AGTCACATCT GTCCTCACCA   2340
   TTGGTCAAGC ACAAACTGCT CTATTACTGG AAATTAACTG GCGTACCGCT TCCTGACGAA   2400
   TGTGACTTCG ACCACCTCAT TATCAGCCGA CAATGGAAGA AAATACTTGA ATCGGCCACT   2460
   CCTGACACTG AGAGGATGAT AAAGCTCGGG CGGGCAGTAC ACCAGACTCT CGACCACCGC   2520
   C                                                                   2521
(2) INFORMATIONS POUR LA SEQ ID NO: 9:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (xi) DESCRIPTION DE LA SEQUENCE: SEQ ID NO: 9:
   CAGACCAAGC TTCTTAAATC CC                                    22
(2) INFORMATIONS POUR LA SEQ ID NO: 10:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: OUI
   (xi) DESCRIPTION DE LA SEQUENCE: SEQ ID NO: 10:
   GTATTCGGGA CAATGC                                           16
(2) INFORMATIONS POUR LA SEQ ID NO: 11:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (xi) DESCRIPTION DE LA SEQUENCE: SEQ ID NO: 11:
   GTGACATCAC TAGCGTCATC C                                     21
(2) INFORMATIONS POUR LA SEQ ID NO: 12:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE : Autre acide nucléique
   (iii) HYPOTHETIQUE: NON
   (iv) ANTI-SENS: OUI
   (xi) DESCRIPTION DE LA SEQUENCE: SEQ ID NO: 12:
   CCGCATCATC AGCGGCCGCG ATCGGTCATG GACAGT 36
(2) INFORMATIONS POUR LA SEQ ID NO: 13:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (xi) DESCRIPTION DE LA SEQUENCE: SEQ ID NO: 13:
   TGACCCTGTC TGGGATGA 18
(2) INFORMATIONS POUR LA SEQ ID NO: 14:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: OUI
   (xi) DESCRIPTION DE LA SEQUENCE: SEQ ID NO: 14:
   GGATCCCGGT CGACACATTG CGGCCGCAAG ATGGGC 36
(2) INFORMATIONS POUR LA SEQ ID NO: 15:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE : NON
   (iv) ANTI-SENS: NON
   (xi) DESCRIPTION DE LA SEQUENCE: SEQ ID NO: 15:
   GACTGGTACC GCGGCCGCAT GCACTTTTTA GGCGGAATTG 40
(2) INFORMATIONS POUR LA SEQ ID NO: 16:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: OUI
   (xi) DESCRIPTION DE LA SEQUENCE: SEQ ID NO: 16:
   TTCGGGACAT TTTCGCGG 18
(2) INFORMATIONS POUR LA SEQ ID NO: 17:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (xi) DESCRIPTION DE LA SEQUENCE: SEQ ID NO: 17:
   TATATGGCGT TAGTCTCC 18
(2) INFORMATIONS POUR LA SEQ ID NO: 18:
   (i) CARACTERISTIQUES DE LA SEQUENCE:
      (A) LONGUEUR: 41 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: OUI
   (xi) DESCRIPTION DE LA SEQUENCE: SEQ ID NO: 18:
   TTGCGAGCTC GCGGCCGCTT ATTACACAGC ATCATCTTCT G                    41
(2) INFORMATIONS POUR LA SEQ ID NO: 19:
   (i) CARACTERISTIQUES DE LA SEQUENCE:
      (A) LONGUEUR: 3116 paires de bases
      (B) TYPE: nucléotide
      (C) NOMBRE DE BRINS: double
      (D) CONFIGURATION: linéaire
   (ii) TYPE DE MOLECULE: ADN (génomique)
   (vi) ORIGINE:
      (A) ORGANISME: Infectious Laryngotracheitis Virus
      (B) SOUCHE: SA-2
   (xi) DESCRIPTION DE LA SEQUENCE: SEQ ID NO: 19:
   GAATTCAATC CTGTTCACCA ACCCCCCGCT ACATTTAATT CTTCACTAGT TTATGTCCTC     60
   ACAAAATATA CACAGCGTCT AAACAACTAC GAGAACCACC AGACGAGATG TGTTAAAGGA    120
   GCTTATTTTG AAAATGAGAC TGTACTCATT TCTCGTCTGA TCCCACTGGC CAAAGAACAG    180
   TATTCGTCAT GGAAGTGGCA GACTGTCTCT TTACATATGG TTTTCCCAGA TCAGAGTTGC    240
   ATTTCCACGG TTATTGTACA TATGTTACTT GCTGACCCGT GCCAGAGGCG AATGTTCGGC    300
   TCTGTCTGCC GCGAGAACGC ATTGCGATTG GATGCATATC ATCTAAACTA CTGGACAGCG    360
   TTTACTTCGA GGCTGATATT ACGGGTGCCA TACACAAAGA TGCAACGGTT TTTGAGGGAA    420
   TTTGAACATG TCCGAGATTG CAAAAGCTTG AACTACGTAG CAGACCCTCT AGGCTTTTGC    480
   ATCTGTAATC CAGGGGTCTT AGTACTGAAA ACACTCGAGA TCGGTTTATA TTTAGCATCG    540
   CTTATTATGT CCACCATGAC ATTGCGGATT TGCTATGATC CGTGTGCATA TATTTTACAT    600
   GAACACGTAA AAATTAGTGC TTGGGTATAT GTAATTGTCT CAGCGGTTCT AGAACTCTTA    660
   TCACTGATGG GTTACACGAC TCCGGCAAAG ACTAAAGTCT CCGCATCGAA ACCTCCCAGT    720
   ATCTTGACTT CATGCCTTGC CAATATTGCT TCGAGCTTAG TCTTGCGTGC ATTGTGCGTG    780
   GCTGCGATTG CGAGCATTGT AATAATTGCA TTTAAATACG AACAGAAGAT ACAAAACAAA    840
   TTGTTCGGGC CTTGAACGGC AATAAAATGT TAAAACACGT TGTGCGGTGT TGTGTCTGAA    900
   TTTGGTCCAT TTGAAGACAG CCCTAGTTCT AACGGCTGAG TATCATTGTT TGAAAGGAGT    960
   AACATCTGGC GGTCAGAATG TACAAAGTAT ACTCGTGGGT GATTTTAGGG CGTGTTCTAC   1020
   TAAGCGGATG TTTGCGGTTT AGACGGCATG CCGCACTACA TTACGGGATT AAGTTACATG   1080
   TCGGATCCGC AGAAGTTCTC GCAGATTATT ATGTCTGCGT GAATATTTCG CACCAACTAC   1140
   TGGATCGCAA TTGTAGTTTG TGGGTTCCAG TGTATGTGTT ATGACGTATA CTGGGAGTGG   1200
   CCCAGACCAT TCAAAAGCCA AGTGGATTAG CTCGCAAACA GCCTGCCAGA TTACTGTCAG   1260
   CTTACCTTTC AGCACCTGCT CCGAGCTTGA CACCTGTTCT GAGTCCGTAG AAGTCTCAAC   1320
   CGTTGTGTAT GCGGTTTCAT TTTTTTTGAC GTGCACGCGG TACATCAACC CACTGACTGC   1380
   GATGGAGACC GGAGAAATCT GGAAGAAATA AAGATAGCGT GAGACACGTT CAGAGCATCT   1440
   TCAATACAAG TACTCATAGC GAAGCGAGAT CAGCAACGGC CCGGACAAAA GATCGCCCTG   1500
   GATGGCCGTT CACGCTGAGG TCAGAATCCC GCCACGAAGC ACATTGTATT CGCCGCAGAT   1560
   CTCATTCTAC AGAAAGTAGC TCCAGATCCC GGTTCATCTG GGCGCGGTAC GTCCAAACGA   1620
   GTCCGTGATT CAAGTGACAG GGGGCGCCGT CGCTGTTCGA ATGGCGAGGC CTCGTTCCCG   1680
   TTACGAGAAC CAGTCAGGTA TGAGCGTACG GTCTTCCATA AGGCGGGTCA GTAGCCCTCG   1740
   AAATAAGTTT ATGAGAGGCC GTGCGCATAT CCAAGTCCGA AGAGGCATCC CCCCTAGACC   1800
   CAGGCGCCGT GCAGGTACAC CAGAAAAGCG ATATAGGGCG CCTATCTTTA CTGTTTCGTT   1860
   GAAGCATTCG CGCAGGTCTT GGGAAAGAAA TCGGGATGAA CTTCGAAGAC CGATTTGGAG   1920
   AGACTTTGTC CGATGCCCAA CGTCATCACG GACCGAAACG AAGGAGTTGC GAAACGTAAC   1980
   ACCGGCCCAA TATTTTGAAA AGGCCGCAAC TGCATTTGGC GGTCTCGGAA AGTGCATTAC   2040
   TGAAGAGTTA AGATTAGAAA ATCAGAAATG TCTTTTAGAC ATGGTAAACC GGGCAGTGGA   2100
   TGATGATGAT TGTGATGATA TCGATCGTGA TAGAGGAATC TGCTTTCCAG CATTTTTGTC   2160
   TTCGGGATCA TCTGACCTTG CAGCCGATGC TGCATTCACT TCGTGGAAGC AGTTTTGTGG   2220
   GCGCGCAGCT TCACTGAAAG GCCGCTGGAC ATCGCGTCCG GATATAGCCA GGTTGGCAAA   2280
   AATTTCACGA GCTGTATATT TGGCGAACTG CTCATTTGAA GAGCTACTTT TTGCATGCGA   2340
   TGAGACACTT GTATGGATGC TTTGGCATCA GTTCGAAGAT GAAAGGATTT ACCCCCACGA   2400
   TCCTATCTTC TCTAACATCT ACTGCGCATG TCAATCTCTA GCCATGCATC TGGGGCCAAT   2460
   CCTGCCGTGT TATCTCTCTA GCATTGGCAG TCAACTAAGA GATACCACTA GATCTCAGGA   2520
   GCTATCACTG AGTAGCGCAA AATGTCCTTT AACTTTATTA CTGACCTTCT TCGACCGGTT   2580
   CTCAAGAATT GTGTATCCGC GATCAGAGGC CATAGTCATG AATCATAAAG CAATAGACCC   2640
   GGCCAGAACA TTGTGGGACA TGTACTATCC TGGGACCTGT TCTAAAAAAA TCCCACTCGT   2700
   TCTGCGCAGC ACGCAAATGT GTGCCGCAAA GAGGAATGCA GAGTGGTTTG TGCGCAGCTC   2760
   TAAGCCGCAG TATACAGTAG GGAAATTTTC TCTTGCGACT TGTTTGACAG TTCTATACAC   2820
   CTATCGACAC ATGGCGGTAT TGTATTGGAA TTGGTGCCAT CCTACGTTTC TGAAGATTGC   2880
   CATCTCTGTG ACTGGTCAGC AGATGGCGCC GCGAGCTCAA TGATGTAAGC GTTCACTGTT   2940
   GCAATCCGGC CTTGTAAATA CACTGTAGTA TTAGCACGGT TGAAAATTTT TAGAATGTTC   3000
   CCCGACTCTC GAAAATTAAC GGAGGTATAA TAAAGAACAC AATACAACGG ACACCGACAA   3060
   CGTGGAGCAT TTTTATTTAG TCTGCAAATC CTTGGTTGAT GAACGTGGTG CCCTGG       3116